(19) **Europäisches Patentamt / European Patent Office / Office européen des brevets**

(11) **EP 1 597 369 B1**

(12) **EUROPEAN PATENT SPECIFICATION**

(45) Date of publication and mention
of the grant of the patent:
**03.10.2007 Bulletin 2007/40**

(21) Application number: **04713540.5**

(22) Date of filing: **23.02.2004**

(51) Int Cl.:
*C12N 15/62* (2006.01)   *C12N 15/70* (2006.01)
*C12N 9/64* (2006.01)   *C07K 14/54* (2006.01)
*C07K 14/56* (2006.01)   *C12P 21/06* (2006.01)

(86) International application number:
**PCT/EP2004/001759**

(87) International publication number:
**WO 2004/074488 (02.09.2004 Gazette 2004/36)**

(54) **USE OF CASPASE ENZYMES FOR MATURATION OF ENGINEERED RECOMBINANT POLYPEPTIDE FUSIONS**

VERWENDUNG VON CASPASEN ZUR HERSTELLUNG VON REIFEN REKOMBINANTEN FUSIONSPROTEINEN

PROCEDE DE PRODUCTION DE PROTEINES FUSIONNEES MATURES EN UTILISANT LES CASPASES

(84) Designated Contracting States:
**AT BE BG CH CY CZ DE DK EE ES FI FR GB GR HU IE IT LI LU MC NL PT RO SE SI SK TR**

(30) Priority: **21.02.2003 EP 03447034**

(43) Date of publication of application:
**23.11.2005 Bulletin 2005/47**

(73) Proprietors:
• **Biotecnol S.A.**
  **2780-920 Oeiras (PT)**
• **Vlaams Interuniversitair Instituut voor Biotechnologie**
  **9052 Zwijnaarde (BE)**
• **Universiteit Gent**
  **9000 Gent (BE)**

(72) Inventors:
• **MERTENS, Nico, Maurice, August, Corneel**
  **B-9120 Melsele (BE)**
• **KELLY, Andrew, Graham**
  **P-2775-680 Carcavelos (PT)**

(74) Representative: **Brants, Johan P.E. et al**
**De Clercq, Brants & Partners cv**
**Edgard Gevaertdreef 10a**
**9830 Sint-Martens-Latem (BE)**

(56) References cited:
**EP-A- 0 207 044**   **EP-A- 0 234 888**
**EP-A- 0 286 239**   **EP-A- 1 013 763**
**WO-A-00/61768**   **WO-A-00/73437**
**WO-A-02/06458**   **WO-A-90/06369**
**WO-A-92/05276**   **WO-A-98/48025**
**WO-A-99/13091**   **WO-A-99/35277**

• **MERTENS NICO ET AL: "New strategies in polypeptide and antibody synthesis: An overview." CANCER BIOTHERAPY & RADIOPHARMACEUTICALS, vol. 19, no. 1, February 2004 (2004-02), pages 99-109, XP008032571 ISSN: 1084-9785**
• **GU ET AL: "Activation of Interferon-gamma Inducing Factor Mediated by Interleukin-1 beta Converting Enzyme" SCIENCE, AMERICAN ASSOCIATION FOR THE ADVANCEMENT OF SCIENCE,, US, vol. 275, no. 5297, 10 January 1997 (1997-01-10), pages 206-209, XP002111766 ISSN: 0036-8075**

SEQUENCE LISTING

<110> Biotecnol S.A. vlaams Interuniversitair instituut voor Biotechnologie universiteit Gent

<120> use of caspase enzymes for maturation of engineered recombinant polypeptide fusions

<130> BTN-001-PCT

<150> EP 03447034.4
<151> 2003-02-21

<160> 63

<170> Patentin version 3.2

<210> 1
<211> 27
<212> DNA
<213> Artificial sequence

<220>
<223> Linker sequence

<400> 1
gggcccggct cggacgaagt ggatatc          27

<210> 2
<211> 8
<212> PRT
<213> Artificial Sequence

<220>

<223> Linker sequence

<400> 2

```
Gly Pro Gly Ser Asp Glu Val Asp
1               5
```

<210> 3
<211> 48
<212> DNA
<213> Artificial sequence

<220>
<223> Linker sequence

<400> 3
gggcccggct cggggggagg cggttcagga ggtgacgaag tggatatc          48

<210> 4
<211> 15
<212> PRT
<213> Artificial sequence

<220>
<223> Linker sequence

<400> 4

```
Gly Pro Gly Ser Gly Gly Gly Gly Ser Gly Gly Asp Glu Val Asp
1               5                   10                  15
```

<210> 5
<211> 48
<212> DNA
<213> Artificial Sequence

<220>
<223> Linker sequence

<400> 5
gggcccggct cgcatcatca ccatcaccat ggagacgaag tggatatc          48

<210> 6
<211> 15
<212> PRT
<213>. Artificial sequence

<220>
<223> Linker sequence

<400> 6

```
Gly Pro Gly Ser His His His His His His Gly Asp Glu Val Asp
1               5                   10                  15
```

17

<210> 7
<211> 48
<212> DNA
<213> Artificial sequence

<220>
<223> Linker sequence

<400> 7
gggcccggct cgcatcacca ccataaggac gatgacgaag tggatatc          48

<210> 8
<211> 15
<212> PRT
<213> Artificial sequence

<220>
<223> Linker sequence

<400> 8


```
Gly Pro Gly Ser His His His His Lys Asp Asp Asp Glu Val Asp
1               5               10                  15
```

<210> 9
<211> 48
<212> DNA
<213> Artificial sequence

<220>
<223> Linker sequence

<400> 9
gggcccgatg acggcgaagg agagaaggac gatgacgaag tggatatc          48

<210> 10
<211> 15
<212> PRT
<213> Artificial sequence

<220>
<223> Linker sequence

<400> 10


```
Gly Pro Asp Asp Gly Glu Gly Glu Lys Asp Asp Asp Glu Val Asp
1               5               10                  15
```

<210> 11
<211> 4
<212> PRT
<213> artificial sequence

<220>

<223> Caspase target sequence

<400> 11

Trp Glu His Asp
1

<210> 12
<211> 4
<212> PRT
<213> Artificial Sequence

<220>
<223> Caspase target sequence

<400> 12

Leu Glu His Asp
1

<210> 13
<211> 4
<212> PRT
<213> Artificial sequence

<220>
<223> Caspase target sequence

<400> 13

Asp Glu Val Asp
1

<210> 14
<211> 4
<212> PRT
<213> Artificial sequence

<220>
<223> Caspase target sequence

<400> 14

Asp Glu His Asp
1

<210> 15
<211> 4
<212> PRT
<213> Artificial sequence

<220>
<223> Caspase target sequence

<400> 15

Val Glu His Asp

<210> 16
<211> 4
<212> PRT
<213> Artificial Sequence

<220>
<223> Caspase target sequence

<400> 16

Leu Glu His Asp

<210> 17
<211> 5
<212> PRT
<213> Artificial sequence

<220>
<223> Caspase target sequence

<400> 17

Asp Glu Val Asp Ser

<210> 18
<211> 4
<212> PRT
<213> Artificial Sequence

<220>
<223> Caspase target sequence

<400> 18

Asp Glu Thr Asp

<210> 19
<211> 4
<212> PRT
<213> Artificial sequence

<220>
<223> Caspase target sequence

<400> 19

Asp Glu Met Asp
1

<210> 20
<211> 4
<212> PRT
<213> Artificial sequence

<220>
<223> Caspase target sequence

<400> 20

Asp Glu Leu Asp
1

<210> 21
<211> 4
<212> PRT
<213> Artificial sequence

<220>
<223> Caspase target sequence

<400> 21

Asp Glu Pro Asp
1

<210> 22
<211> 4
<212> PRT
<213> Artificial Sequence

<220>
<223> caspase target sequence

<400> 22

Asp Glu Ala Asp
1

<210> 23
<211> 4
<212> PRT
<213> Artificial Sequence

<220>
<223> Caspase target sequence

<400> 23

```
Asp Glu Gln Asp
1
```

<210> 24
<211> 4
<212> PRT
<213> Artificial Sequence

<220>
<223> Caspase target sequence

<400> 24

```
Asp Thr Val Asp
1
```

<210> 25
<211> 4
<212> PRT
<213> Artificial Sequence

<220>
<223> Caspase target sequence

<400> 25

```
Asp Gln Thr Asp
1
```

<210> 26
<211> 4
<212> PRT
<213> Artificial sequence

<220>
<223> caspase target sequence

<400> 26

```
Asp Ile Thr Asp
1                     .
```

<210> 27
<211> 4
<212> PRT
<213> Artificial Sequence

<220>
<223> Caspase target sequence

<400> 27

Asp Met Gln Asp
1

<210> 28
<211> 4
<212> PRT
<213> Artificial Sequence

<220>
<223> caspase target sequence

<400> 28

Asp Ser Leu Asp
1

<210> 29
<211> 4
<212> PRT
<213> Artificial sequence

<220>
<223> Caspase target sequence

<400> 29

Asp Gly Pro Asp
1

<210> 30
<211> 4
<212> PRT
<213> Artificial sequence

<220>
<223> Caspase target sequence

<400> 30

Asp Val Pro Asp
1

<210> 31
<211> 4
<212> PRT
<213> Artificial sequence

<220>
<223> caspase target sequence

<400> 31

Asp Arg His Asp
1

<210> 32
<211> 4
<212> PRT
<213> Artificial Sequence

<220>
<223> Caspase target sequence

<400> 32

Asp His Val Asp
1

<210> 33
<211> 4
<212> PRT
<213> Artificial Sequence

<220>
<223> Caspase target sequence

<400> 33

Asp Pro Ser Asp
1

<210> 34
<211> 4
<212> PRT
<213> Artificial Sequence

<220>
<223> Caspase target sequence

<400> 34

Asp Ser Val Asp
1

<210> 35
<211> 4
<212> PRT
<213> Artificial Sequence

<220>
<223> Caspase target sequence

<400> 35

```
                            Asp Ser Leu Asp
                            1
```

<210> 36
<211> 4
<212> PRT
<213> Artificial Sequence

<220>
<223> Caspase target sequence

<400> 36

```
                            Asp Ser Tyr Asp
                            1
```

<210> 37
<211> 4
<212> PRT
<213> Artificial Sequence

<220>
<223> Caspase target sequence

<400> 37

```
                            Asp Gln Thr Asp
                            1
```

<210> 38
<211> 4
<212> PRT
<213> Artificial sequence

<220>
<223> caspase target sequence

<400> 38

```
                            Asp Asp Val Asp
                            1
```

<210> 39
<211> 4
<212> PRT
<213> Artificial sequence

<220>
<223> Caspase target sequence

<400> 39

Asp Ala Gly Asp
1

<210> 40
<211> 4
<212> PRT
<213> Artificial sequence

<220>
<223> Caspase target sequence

<400> 40

Met Glu Leu Asp
1

<210> 41
<211> 4
<212> PRT
<213> Artificial sequence

<220>
<223> Caspase target sequence

<400> 41

Val Glu Val Asp
1

<210> 42
<211> 4
<212> PRT
<213> Artificial Sequence

<220>
<223> caspase target sequence

<400> 42

Leu Glu Val Asp
1

<210> 43
<211> 4
<212> PRT
<213> Artificial sequence

<220>
<223> Caspase target sequence

<400> 43

Leu Gln Thr Asp
1

<210> 44
<211> 4
<212> PRT
<213> Artificial sequence

<220>
<223> Caspase target sequence

<400> 44

Val Ser Trp Asp
1

<210> 45
<211> 4
<212> PRT
<213> Artificial sequence

<220>
<223> caspase target sequence

<400> 45

Tyr Val Pro Asp
1

<210> 46
<211> 4
<212> PRT
<213> Artificial Sequence

<220>
<223> Caspase target sequence

<400> 46

Ser His Val Asp
1

<210> 47
<211> 4
<212> PRT
<213> Artificial sequence

<220>
<223> caspase target sequence

<400> 47

His Leu Ala Asp
1

<210> 48
<211> 4
<212> PRT
<213> Artificial sequence

<220>
<223> Caspase target sequence

<400> 48

Ala Arg Gln Asp
1

<210> 49
<211> 4
<212> PRT
<213> Artificial sequence

<220>
<223> Caspase target sequence

<400> 49

Pro Ala Pro Asp
1

<210> 50
<211> 4
<212> PRT
<213> Artificial sequence

<220>
<223> Caspase target sequence

<400> 50

Ser Ser Thr Asp
1

<210> 51
<211> 4
<212> PRT
<213> Artificial Sequence

<220>
<223> Caspase target sequence

<400> 51

Leu Gly Thr Asp
1

<210> 52
<211> 4
<212> PRT
<213> Artificial sequence

<220>
<223> Caspase target sequence

<400> 52

Leu Ser Val Asp
1

<210> 53
<211> 4
<212> PRT
<213> Artificial Sequence

<220>
<223> Caspase target sequence

<400> 53

Ala Leu Asp Asp
1

<210> 54
<211> 4
<212> PRT
<213> Artificial sequence

<220>
<223> Caspase target sequence

<400> 54

Leu Ser Ser Asp
1

<210> 55
<211> 4
<212> PRT
<213> Artificial Sequence

<220>
<223> Caspase target sequence

<400> 55

Ala Leu Ala Asp
1

<210> 56
<211> 4
<212> PRT
<213> Artificial Sequence

<220>
<223> Caspase target sequence

<400> 56

Phe Ile Gln Asp
1

<210> 57
<211> 4
<212> PRT
<213> Artificial Sequence

<220>
<223> Caspase target sequence

<400> 57

Asp Asp Asp Lys
1

<210> 58
<211> 4
<212> PRT
<213> Artificial Sequence

<220>
<223> Caspase target sequence

<400> 58

Arg Gly Pro Arg
1

<210> 59
<211> 183
<212> PRT
<213> Mus musculus

<400> 59

Ala Leu Ala Asp
1

```
Met Gly Ser Pro Leu Pro Ile Thr Pro Val Asn Ala Thr Cys Ala Ile
1               5                  10                  15

Arg His Pro Cys His Gly Asn Leu Met Asn Gln Ile Lys Asn Gln Leu
            20              25                  30

Ala Gln Leu Asn Gly Ser Ala Asn Ala Leu Phe Ile Ser Tyr Tyr Thr

        35                  40                  45

Ala Gln Gly Glu Pro Phe Pro Asn Asn Val Glu Lys Leu Cys Ala Pro
        50              55                  60

Asn Met Thr Asp Phe Pro Ser Phe His Gly Asn Gly Thr Glu Lys Thr
65                  70                  75                  80

Lys Leu Val Glu Leu Tyr Arg Met Val Ala Tyr Leu Ser Ala Ser Leu
                85                  90                  95

Thr Asn Ile Thr Arg Lys Asp Gln Lys Val Leu Asn Pro Thr Ala Val
                100                 105                 110

Ser Leu Gln Val Lys Leu Asn Ala Thr Ile Asp Val Met Arg Gly Leu
            115                 120                 125

Leu Ser Asn Val Leu Cys Arg Leu Cys Asn Lys Tyr Arg Val Gly His
        130                 135                 140

Val Asp Val Pro Pro Val Pro Asp His Ser Asp Lys Glu Ala Phe Gln
145                 150                 155                 160

Arg Lys Lys Leu Gly Cys Gln Leu Leu Gly Thr Tyr Lys Gln Val Ile
                165                 170                 175

Ser Val Val Val Gln Ala Phe
                180
```

<210> 60
<211> 165
<212> PRT
<213> Homo sapiens

<400> 60

```
Met Cys Asp Leu Pro Gln Thr His Ser Leu Gly Ser Arg Arg Thr Leu
 1               5                  10                  15

Met Leu Leu Ala Gln Met Arg Arg Ile Ser Leu Phe Ser Cys Leu Lys
            20                  25                  30

Asp Arg His Asp Phe Gly Phe Pro Gln Glu Glu Phe Gly Asn Gln Phe
            35                  40                  45

Gln Lys Ala Glu Thr Ile Pro Val Leu His Glu Met Ile Gln Gln Ile
        50                  55                  60

Phe Asn Leu Phe Ser Thr Lys Asp Ser Ser Ala Ala Trp Asp Glu Thr
65                  70                  75                  80

Leu Leu Asp Lys Phe Tyr Thr Glu Leu Tyr Gln Gln Leu Asn Asp Leu
                85                  90                  95

Glu Ala Cys Val Ile Gln Gly Val Gly Val Thr Glu Thr Pro Leu Met
            100                 105                 110

Lys Glu Asp Ile Leu Ala Val Arg Lys Tyr Phe Gln Arg Ile Thr Leu
            115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165
```

<210> 61
<211> 220
<212> PRT
<213> mammalian

<400> 61

Ser Pro Ile Leu Gly Tyr Trp Lys Ile Lys Gly Leu Val Gln Pro Thr
1               5               10                      15

Arg Leu Leu Leu Glu Tyr Leu Glu Glu Lys Tyr Glu Glu His Leu Tyr
            20              25                      30

Glu Arg Asp Glu Gly Asp Lys Trp Arg Asn Lys Lys Phe Glu Leu Gly
        35              40                      45

Leu Glu Phe Pro Asn Leu Pro Tyr Tyr Ile Asp Gly Asp Val Lys Leu
    50                  55                      60

Thr Gln Ser Met Ala Ile Ile Arg Tyr Ile Ala Asp Lys His Asn Met
65                  70              75                      80

Leu Gly Gly Cys Pro Lys Glu Arg Ala Glu Ile Ser Met Leu Glu Gly
                85                  90                      95

Ala Val Leu Asp Ile Arg Tyr Gly Val Ser Arg Ile Ala Tyr Ser Lys
            100                 105                 110

Asp Phe Glu Thr Leu Lys Val Asp Phe Leu Ser Lys Leu Pro Glu Met
        115                 120                 125

Leu Lys Met Phe Glu Asp Arg Leu Cys His Lys Thr Tyr Leu Asn Gly
    130                 135                 140

Asp His Val Thr His Pro Asp Phe Met Leu Tyr Asp Ala Leu Asp Val
145                 150                 155                 160

Val Leu Tyr Met Asp Pro Met Cys Leu Asp Ala Phe Pro Lys Leu Val
                165                 170                 175

Cys Phe Lys Lys Arg Ile Glu Ala Ile Pro Gln Ile Asp Lys Tyr Leu
            180                 185                 190

Lys Ser Ser Lys Tyr Ile Ala Trp Pro Leu Gln Gly Trp Gln Ala Thr
        195                 200                 205

Phe Gly Gly Gly Asp His Pro Pro Lys Ser Asp Leu
    210             215                 220

<210> 62
<211> 111
<212> PRT
<213> Escherichia coli

<400> 62

```
Ser Asp Lys Ile Ile His Leu Thr Asp Asp Ser Phe Asp Thr Asp Val
1               5                   10                  15

Leu Lys Ala Asp Gly Ala Ile Leu Val Asp Phe Trp Ala Glu Trp Cys
            20              25                  30

Gly Pro Cys Lys Met Ile Ala Pro Ile Leu Asp Glu Ile Ala Asp Glu
            35              40              45

Tyr Gln Gly Lys Leu Thr Val Ala Lys Leu Asn Ile Asp Gln Asn Pro
    50              55                  60

Gly Thr Ala Pro Lys Tyr Gly Ile Arg Gly Gly Ile Pro Thr Leu Leu
65              70                  75                      80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser
            100             105             110
```

<210> 63
<211> 4
<212> PRT
<213> Artificial sequence

<220>
<223> Caspase target sequence

<220>
<221> MISC_FEATURE
<222> (2)..(3)
<223> X = any amino acid

<400> 63

```
Asp Xaa Xaa Asp
1
```

## Description

### Field of the invention

The invention relates generally to the field of molecular biology, biotechnology or process engineering for the production of proteins or peptides. The invention relates to methods of producing and isolating recombinant proteins using fusion protein constructs comprising specific recognition sites for a maturating protease. The invention specifically relates to the use of caspase proteases for the maturation of engineered recombinant fusion proteins or polypeptides. These molecules are engineered using recombinant DNA technology to comprise a specific target sequence for a caspase between a first fusion part and the polypeptide of interest. After processing, the desired mature format of the protein or polypeptide is obtained.

### Background of the invention

The heterologous expression of recombinant proteins is hampered by several technological difficulties. The expression level can be compromised due to a variety of causes.

First, the level of translation can be hampering a reasonable protein production level. This needs to be engineered and optimized for each individual protein, which is a time-consuming task. Second, the protein of interest can be unstable. This instability can be due to several causes. It has been described that the N-terminus of a protein is determining the overall stability of the protein, which will determine its likelihood of being degraded by host proteases. Third, despite high yield of expression of recombinant proteins, the major setback of producing recombinant proteins in *E. coli* is the formation of insoluble precipitates of the expressed protein. These "inclusion bodies" are in most cases biologically inactive. Therefore, a refolding process is required to regain soluble protein that is biologically active. This is a cumbersome process, and implementation in a production environment is raising the cost of the process considerably. The refolding process is by no means a guarantee of full biological activity of the protein. Isomers can be formed with the same biophysical characteristics, but with a much lower biological activity. Usually, these folding isomers have a different hydrophobic surface and can be identified by reverse phase chromatography. Furthermore, when producing mature proteins in the cytoplasm of E. coli, the removal of the obligatory N-terminal methionine amino acid might not be as efficient, resulting in a non-controlled heterogenicity in the protein preparation. The N-terminal methionine is also formylated in E. coli, which is unwanted as it is a specific immunogenic signal in human and animal.

These problems can be overcome by adapting a fusion protein strategy. Fusion proteins are extensions of the protein of interest with one or more amino acids. Fusion proteins can be constructed in order to couple a peptide tag to the protein of interest, or to create a fusion with a larger polypeptide. A preferred strategy is to fuse the additional polypeptide part at the N-terminus of the polypeptide of interest. Advantages of using this fusion protein strategy include the fact that with a proper fusion partner, expression is generally high and need no further optimization, as the N-terminus can be chosen to stabilize the fusion protein from degradation, whereby the fusion protein is generally better protected against protease degradation, and solubility of the fusion construct is often improved. Furthermore, a specific simple purification method can be designed targeting the fusion part, and allowing the fusion protein to be isolated in a simple chromatographic step. If such a purification step does not exists for the fusion part, affinity "tags" can be fused to the fusion part. The newly composed fusion part then has acquired specific characteristic behaviors on an adsorption column. Examples are peptide tags recognized by specific antibodies, peptide tags with an affinity towards a certain protein, protein domains with an affinity towards another protein or protein domain, specifically isolated binding peptides, protein domains or proteins, and proteins or domains thereof that specifically bind a non-pertinacious substrate. One or more of these affinity tags may be combined in the fusion protein.

For this purpose fusion partners have been identified empirically, on the basis on how well they are produced in *E. coli,* or on the basis of their hydrophilicity. Examples of empirically identified partners increasing the soluble expression of the protein fused to the polypeptide of interest are GST, thioredoxin or MBP. Examples of fusion partners chosen on the basis of their high hydrophilic character are NusA and GrpE. The result, however, is still unprecictable, depending on the intrinsic properties of the protein that is expressed. Examples of the affinity tags mentioned are polyhistidine tags, poly-arginine tags, peptide substrates for antibodies, chitin binding domain, the RNase S peptide, maltose binding protein, gluthathion S-transferase, protein A, beta-galactosidase.

Although interesting, the fusion protein approach shifts the bottleneck of the production process to a different level: the removal of the fusion part and maturation of the protein of interest.

In order to obtain a mature protein of interest starting from the fusion protein, the fusion partner or the composed fusion part must be removed from the protein of interest. To this end, several methods have been described. Chemical cleavage methods have been devised for cleavage after specific amino acids. However, the amino acid preceding the first amino acid of the mature part of the protein of interest should then be unique, or at least not occur in the protein of interest. Also, the harsh chemical conditions needed for the induction of this cleavage can lead to chemical protein degradation: oxidation, deamidation, cyclic imide formation, iso-aspartate formation are just examples of this. Chemical protein degradation will lead to heterogeneity in the final protein preparation; it may introduce antigenic determinants in the protein or diminish its activity, making it less suitable for use as a therapeutic substance. Proteases have been described that have some specificity towards certain peptide sequences. In order to use these proteases a linker is introduced between the fusion part and the protein of interest, to compose a fusion protein of the formula

$$F_l - L - P$$

Where F represents the fusion part that can be composed of different units $F_1$, $F_2$,..$F_n$, L is a linker sequence that contains a recognition sequence for the protease used and possibly also a spacer to increase the chance of the protease site to be accessible and P is the protein of interest.

Some of the proteases used for this purpose are cutting within the linker, as their specificity is determined both by the upstream sequence of the cleavage site ($P_1$ positions, numbered away from the cleavage site towards the N-terminus) and by the downstream sequence of the cleavage site (P', positions, numbered away from the cleavage site towards the C-terminus). Processing with these enzymes will not result in obtaining a mature protein, but in a protein still fused to a number of non-native amino acids. While for a number of biochemical purposes this might be well acceptable, in the development of a therapeutic reagent it is highly desirable to obtain but the mature sequence of the desired protein of peptide of interest. Examples of such proteases and examples of the cutting sequences are:

| ENZYME | SEQUENCE |
|---|---|
| IgA protease | Thr-Pro-Ala-Pro-Arg-Pro-Pro|^|Thr-Pro |
| Collagenase | Pro-Xaa|^|Gly-Pro |
| HRV3C | Leu-Glu-Val-Leu-Phe-Gln|^|Gly-Pro |
| TEV | Glu-Xaa-Xaa-Tyr-Gin|^|(Gly/Ser) |

The notation '|^|' refers to the position in the sequence where the protease cuts.
Other enzymes show no or very little preference for the $P'_1$ and $P'_2$ position, and thus allow maturation of the fusion protein to obtain the protein of interest, without any artificial amino acids fused to it. Obviously, for obtaining material for therapeutic use in human and animal, this is the most preferred option.
Examples of such restricted proteases and an example of the cutting sequence with no specificity C-terminal to the cleavage point are:

| ENZYME | SEQUENCE |
|---|---|
| Trypsin | Arg|^|, Lys|^| |
| Thrombin | Arg-Gly-Pro-Arg|^| |
| Factor Xa | Ile-Glu-Gly-Arg|^| |
| Enterokinase | Asp-Asp-Asp-Lys|^| |

Enzymes like Trypsin obviously have only a very limited use, since there is a high chance that they also degrade the protein of interest.

The specificity of Thrombin is also low. Optimum cleavage sites for Thrombin are given below. In the first three examples, the formula for the recognition site is
P4-P3-P-R/K |^| P1'-P2'
where P3 and P4 are hydrophobic amino acids and P1' and P2' are non-acidic amino acids. The bond behind R/K is cleaved. Possible cleavage sites of thrombin are however variable (one letter code for amino acids is shown):

| | P4 | P3 | P2 | P1 | P1' | P2' |
|---|---|---|---|---|---|---|
| 1 | M | Y | P | R | G | N |
| 2 | I | R | P | K | L | K |
| 3 | L | V | P | R | G | S |
| 4 | | | A | R | G | |
| 5 | | | G | K | A | |

In the cases 4 and 5, shown above, the cleavage site is less defined and sites similar to these sequences will be regularly found in natural proteins. Thrombin therefore seems to be only little more specific than Trypsin. For this reason, Thrombin cleavage will also have a high change of hydrolysing the protein of interest.

The specificity of Factor Xa is less stringent and examples are known where the protein of interest is cleaved at unsuspected sites, and specificity is not higher than the one observed with Thrombin. This often leads to processing and degradation of the protein of interest. Further degradation of the clipped protein parts is often due to activity of contaminating proteases. Moreover, the production of Factor Xa is rather difficult, since it needs to be produced in a mammalian cell-based expression system and it requires post-productional activation steps (activation by Russell viper venom). This makes the enzyme not the best choice for large-scale production since cost of enzyme might outrun the cost of production of the fusion protein comprising the protein of interest.

Apart from the lack of specificity of these proteases, the efficiency of processing and the yield of mature protein obtained is rather low, and often insufficient to consider the development of a production process using these enzymes.

Bovine Enterokinase is an enzyme with a higher degree of specificity, but several cases are known where the enzyme did not cleave the protein of interest or degraded the protein of interest in an unexpected manner. Moreover, the Enterokinase or its catalytic domain is difficult to produce and purify.

Use of a caspase enzyme for the maturation of engineered recombinant polypeptide fusions has been reported in the prior art.

WO 00/61768 for instance is directed to the preparation of biologically active molecules from their biologically inactive precursors by mutating their native cleavage site to a site capable of being cleaved by a protease and cleavage of the mutated molecule to yield a biologically active molecule. In this document, a cDNA encoding a mutated precursor of pro-IL-18$_{LETD}$ protein, optionally fused to a glutathione-S-transferase (GST) coding sequence and downstream of an IPTG inducible tac promoter, is disclosed. This document further discloses the expression and purification of GST-proIL-18$_{LETD}$ fusion protein, the binding of the inactive precursor protein to gluthatione-agarose beads and its cleavage using a caspase-8 (group III), to produce human IL-18.

EP 1 013 763 A1 discloses canine interleukin 18 and a canine interleukin 1-beta converting enzyme (ICE, caspase-1) for the treatment of canine immunological diseases. Canine interleukin 1-beta convertase can cleave precursor forms of interleukin 18 and 1 beta to give the active forms.

In another example, Gu et al. (1997, Science, vol. 275, pages 206 to 209) discloses the activation of the inactive IL-1-beta precursor to the pro-inflammatory cytokine by interleukin-1-beta converting enzyme (ICE, caspase -1). ICE was shown to cleave the precursor of interferon-gamma inducing factor (IGIF) at the authentic processing site, thereby activating IGIF and facilitating its export. Co-expression of proIGIGF with ICE or its homolog (caspase-4) was shown to result in the cleavage of proIGIF into the polypeptide similar in size to the naturally occurring 18-kD IGIF. Moreover, co-expression with a caspase-3 (group II, CPP32) resulted in the cleavage of proIGIF into a 14-kd polypeptide.

In addition, polynucleic acids that encode fusion proteins, which comprise a polypeptide, a linker sequence with caspase recognition site and a polypeptide of interest, have also been disclosed in the art.

For instance, WO 02/06458 discloses a purified polypeptide having luciferase activity and a recognition site specifically cleavable by a caspase. More in particular, this document discloses a polynucleic acid encoding a protein comprising a) an aminoterminal polypeptide (a targeting sequence), b) a linker sequence comprising a caspase group II recognition site (in particular a cleavable linker comprising the DEVD and/or DEHD site) and c) the polypeptide of interest (luciferase). The polypeptide is used as a reporter construct, where cleavage results in a decrease in luciferase activity.

In another example WO 00/73437 discloses a polynucleic acid encoding a fusion protein comprising a sequence of amino acids in which GFP is engineered to contain a group II caspase site. The (linker) sequence comprises at least one caspase cleaving site and examples thereof include caspase group II recognition sites (e.g. DXXD, DEVD, DEHD, DELD). The fusion proteins are used for identifying activators or inhibitors of caspases in living cells or *in vitro.*

## Summary of the invention

The present invention uses a process to obtain mature protein, a protein domain or a peptide starting with any amino acid of choice at its N-terminus by producing it as a fusion Protein to an N-terminal fusion partner via connection with an engineered linker sequence and thereafter releasing it in a specific way by incubation with a protease that belongs to the caspase family of cysteine proteases. The invention also relates to the design of peptide linkers used to connect a fusion partner to the mature protein whereby said linker is specifically digested by such a caspase. The invention further relates to the expression and purification of fusion proteins comprising a fusion part and a mature protein or peptide of interest comprising a linker sequence designed to be processed by a caspase protease. This invention can be used to facilitate production of a recombinant protein in a functional and/or mature form and to facilitate recovery processes.

This invention describes the use of caspases as maturation proteases for recombinant fusion proteins containing an engineered caspase recognition site in the linker connecting the fusion protein and the protein of interest. The present invention describes a process using caspases to separate the fusion part and the linker sequence from a protein of interest without limitation of choice of the first amino add of the mature protein, making the method suitable for producing human and animal therapeutics. Also, the caspase enzymes can be produced in an efficient and cheap way in an *E. coli* expression system and can be subsequently purified leaving no traces of host proteases. These characteristics make the invention suitable for process development of the production of peptides, proteins or part of proteins at large scale.

The present invention results in a method that cleaves the fusion part and the linker sequence with high specificity, and several specificities can be engineered In the linker depending on the caspase used. Surprisingly, it was found that even proteins with suspected cleavage sites on the basis of their protein sequence were only cut at the engineered site and not at cryptic Internal sites. Furthermore, cleavage times can be as short as several minutes to one hour. The enzyme can be specifically removed from the processing reaction and several protease inhibitors are known to the person skilled in the art, which allow to end the processing reaction in a controlled way.

In one embodiment, the present invention relates to a method as defined in claim 1 for producing a polypeptide of interest in a biologically active form, in a suitable host, preferably a prokaryote, more preferably *Escherichia coli*.

In a further embodiment, the invention relates to a method as defined in claim 1 wherein said caspase recognition site comprises the amino acid sequence DEDL (SEQ ID NO 20) DXXD (SEQ ID NO 63). preferably DEVD (SEQ ID NO 13) or DEHD (SEQ ID NO 14).

The invention further relates to any of the methods defined above wherein said caspase is selected from the group consisting of caspase-2, caspase-3, caspase-7, CED-3, reversed caspase-3 and reversed caspase-7.

According to a further embodiment, the invention relates to any of the methods defined above wherein the linker sequence In the fusion protein comprises at least 4, preferably at least 5, 6, 7, 8, 9, 10, 11, 12, 13, 14, 15, 16, 17, 18. 19 or 20 amino acids, more preferably at least 25, 30 or 40 amino acids; preferably said linker sequence is represented by any of the SEQ ID NOs as defined in claim 8.

According to a preferred embodiment of the methods of the invention, the caspace recognition site of the fusion protein comprises the amino acid sequence as defined in claim 7.

The invention further relates to a method as defined above wherein the linker sequence as defined above comprises at least 2, 3, 4 or 5 hydrophilic, or at least 2, 3, 4 or 5 charged, or at least 2, 3. 4 or 5 small amino acids.

According to a further embodiment, the invention relates to a method as defined above wherein said cleaving of the fusion protein by the caspase is carried out in vitro; preferably a yield of at least 80% of mature polypeptide of interest is obtained within 60 minutes as measured or judged by densitometric scanning.

In another embodiment, the Invention relates to a method according to any of the methods described above further characterized in that the caspase protein is produced in the same host as the fusion protein; preferably, the caspase protein sequence is comprised in, i.e. is part of, the sequence of the fusion protein.

In one embodiment, in the methods of the invention, the production (and/or the induction of the production) of the caspase protein and the production (and/or the induction of the production) of the fusion protein are sequential, preferably, the caspase protein is produced (and/or the production of the caspase protein is induced) after the production (and/or the induction of the production) of the fusion protein.

The invention further relates to any of the methods described above wherein said cleaving of the fusion protein is carried out in vivo.

According to another embodiment, the invention relates to a method for producing a polypeptide of interest in a biologically active (and/or mature) form as defined in claim 16.

According to a further embodiment the invention relates to a method for producing a polypeptide of interest in a biologically active (and/or mature) form as defined in claim 17.

The invention further relates to any of the methods described above wherein a single polynucleic acid encodes the fusion protein and the caspase, or, in the alternative, wherein separate polynucleic acids encode the fusion protein and the caspase.

According to yet another embodiment, the invention relates to a polynucleic acid encoding any of the fusion proteins ascribed above. More particularly, the invention relates to a polynucleic acid as defined in claim 22.

The invention relates to a polynucleic acid as described above, encoding a caspase protein. Preferably said caspase protein is selected from the group consisting of caspase-2, caspase-3, caspase-7, CED-3, reversed caspase-3 and reversed caspase-7.

According to other embodiments of the invention, the aminoterminal polypeptide encoded by the polynucleic acids of the invention preferably is selected from the group comprising, but not limited to, glutathione S-transferase gene GST. E. coli thioredoxin TRX, NusA, chitin binding domain CBD, chloramphenicol acetyl transferase CAT, protein A (prot A). LysRS, maltose binding protein (MBP), ubiquitin, calmodulin, DsbA. DsbC and lambda gpV.

Preferably, the linker sequence of the polynucleic acid of the invention comprises at least 5, preferably at least 6, 7, 8, 9, 10. 11, 12, 13, 14, 15, 16, 17, 18, 19 or 20 amino acids, more preferably at least 25, 30 or 40 amino acids; preferably said linker sequence is represented by any of SEQ ID NOs 2, 4, 6, 8 or 10, 13, 14, 20 or 63.

According to another embodiment, said linker sequence consists of 4 amino acids, comprising a caspase recognition site consisting of the amino acid sequence as defined in claim 26.

The invention further relates to any of the polynucleic acids described above wherein the expression of said fusion protein and/or said caspase protein is under the control of a suitable promoter, preferably said promoter is an inducible promoter, and even more preferred, the fusion protein and the caspase protein are under the control of a different inducible promoter.

The invention further relates to any of the polynucleic acids described above wherein said fusion protein further comprises a detection or affinity tag, preferably said detection or affinity tag is selected from the group consisting of a polyhistidine tag, a polyarginine tag, a streptavidin binding tag, a biotinylated tag and a tag recognized by an antibody.

The invention also relates to a vector comprising any of the polynucleic acids described above, and to a host cell comprising said vector or any of said polynucleic adds.

The invention further relates to a fusion protein encoded by the nucleic adds described above, and to an antibody specifically binding to said fusion protein.

The invention further relates to the use of any of the polynucleic acids, vectors, host cells or fusion proteins described above, for the production of a polypeptide of interest in a mature and/or biologically active form.

The invention further relates to a bioreactor suitable for the production of biologically active, i.e. functional, and/or mature proteins of interest as defined in claim 36. Preferably the substrate is contained within an affinity column.

According to another embodiment, the invention relates to a bioreactor suitable for the production of biologically active, i.e. functional, and/or mature proteins of interest as defined in claim 37.

The invention also relates to a method for producing a protein of interest as defined in claim 38.

Preferably the substrate is contained within an affinity column.

## Detailed description of the invention

Surprisingly we found that a highly specific and highly efficient processing can be obtained by the use of caspases as specific proteases, when using a specially designed fusion protein as substrate.

Caspases are cystein proteases that specifically cleave after an aspartyl residue. The caspases gene family thus far contains at least 14 mammalian members, of which at least 11 human enzymes are known. A first classification can be made after phylogenetic analysis and divides the family In two classes: the Caspase-1 (ICE) related enzymes and the Caspase-3 (CED-3) related enzymes. A further division can be made on the basis of short (caspase-3. -6 and -7), or long (caspase-1, -2, -4, -5, and caspases-8 to 14) prodomains. Alternatively, the proteases can be subdivided on the basis of their substrate specificity. This would divide the caspases in three different groups. Group I caspases (Caspases 1, 4, 5, 13) are liberal for substitutions at the P4 position but prefer bulky hydrophobic amino acids such as Tyr or Trp. Group II caspases (Caspases 2, 3, 7) are substantially more stringent in S4, requiring a P4 Asp. The preferred cleavage motif for this group is DEXD, but many substrates of the formula DXXD have been identified. Caspase-2 has even stringency at position P5. Table I gives a list of non-limiting examples of natural substrate proteins. It can be noted that there is a high preference for small hydrophilic amino acids at the P1' position of the natural substrates and that the consensus sequence is not met by a large group of target sequences. It should also be remembered that not all targets are cleaved with the same efficiency. Group III caspases (Caspases 6, 8, 9, 10) prefer branched chain aliphatic amino acids in P4.

Fusion protein strategies for enhancing expression level, improving solubility and facilitating purification of the protein have been around since 1983 and before. None of these strategies has been used in a large-scale production process. Obviously, the bottleneck of adapting a fusion protein strategy for large-scale process development is in the specificity, activity, availability and purity of the protease enzyme used. The specificity needs to be high enough to at least allow a number of proteins to be cleaved only at the engineered cleavage site in the connecting linker sequence. The activity of the enzyme needs to be high enough to allow sufficient cleavage in a short period of time. This will avoid hold-up time during the production and minimizes degradation of the protein of interest during incubation. The protease needs to be available at low cost, so an efficient expression system and a low-cost production method are necessary. Therefore, an engineered protease containing an affinity tag might be a good solution. The protease should also be sufficiently pure, especially free of even trace contamination of non-specific proteases from the host organism. Since no protease will fit these requirements for all possible proteins of interest, industry will need a number of proteases to be developed for use in such a process. These proteases will preferably have a different specificity. A case-by-case screening can determine the optimal processing strategy for each polypeptide of interest.

This invention relates to the use of caspase activity to cut an artificial fusion protein, comprising a first part comprising a protein or peptide as a fusion part, a second part comprising a linker artificially designed to maximize cutting with the enzyme, and a third part comprising the protein or peptide of interest to be isolated in a mature and functional form.

The caspase protein itself can be part of a fusion protein with the intention to e.g. optimize expression level or solubility of the caspase enzyme, or to optimize the easy recovery of the caspase enzyme, or to enable the caspase enzyme to be coupled efficiently to a solid support.

The first steps in the elaboration of the processing consist of choosing a caspase, followed by designing, choosing or selecting a linker sequence that is cut by the caspase of choice.

Cloning of Caspases

Gene sequences of caspases genes can be found at the National Centre for Biotechnology Information (http: //www.ncbi.nlm.nih.gov/). Caspases can be cloned from cDNA using appropriate primers hybridizing at the beginning and at the end of the coding sequence. Using the PCR reaction, an amplified DNA can be obtained and cloned in a suitable plasmid. Alternatively, the cDNA library is cloned and seeded to single colonies. The colony lysates can then be probed with a marked oligonucleotide that hybridizes specifically with the caspase gene. These techniques are known to a person skilled in the art.

Designing a Caspase digestible linker in a fusion protein

Preferably the linker sequence comprises a target sequence of a natural occurring substrate of the caspases. Most preferably, the linker comprises the most efficiently cut target sequence known or isolated for the caspase used. It is possible that this sequence is not a naturally occurring target sequence, but derived from a consensus sequence, or isolated by a screening method, or selected from a biological enrichment method, or obtained after a biological selection method.

Table I gives examples of target sequences obtained by comparing natural target sequences, or by synthesising sequences isolated by random library screening. The optimized sequences may also be obtained by comparing specific enzyme activity of the caspase on a peptide substrate.

Some preferred target sequences are:

| Caspace Group | Caspases | Preferred sequence |
|---|---|---|
| Group I | 1, 4, 5, 13 | WEHD (SEQ ID NO 11) LEHD (SEQ ID NO 12) |
| Group II | 2, 3, 7, CED-3 | DEVD (SEQ ID NO 13) DEHD (SEQ ID NO 14) |
| Group III | 6, 8, 9, 10, | VEHD (SEQ ID NO 15) LEHD (SEQ ID NO 16) |

Preferably, the linker sequence further comprises a spacer region to increase the space between the fusion partner F and the caspase cutting site in the linker. Most preferably, the linker is designed or optimized in such a way, that the caspase target sequence is easily accessible by the caspase enzyme.

The linker sequence can be further optimized in order to increase the solubility of the fusion product. This can be done, as a non-limiting example, by enriching the sequence with hydrophilic amino acids, or with charged amino acids. The current state of the art makes it possible to design experiments in order to select linker sequences that would improve the solubility by screening or selecting a number of variants. These variants may be a defined number of options derived from a calculated choice, or derived from a randomized library of sequences.

The linker sequence can also be further optimized to comprise sequences that are recognized by a ligand or antibody in order to serve as an affinity target in an affinity based separation step, or to be used for easy and sensitive detection of the protein incorporating the sequence. Also, the linker may contain a high concentration of a certain class of amino acids, in order to improve adsorption to non-specific chromatography columns, such as ion exchange, hydrophobic interaction, ligand binding.

Construction of a fusion protein

The fusion construct comprises a combination of the general formula

$$\textbf{F-L-G}$$

where F is a fusion partner of choice, L is the linker sequence chosen, and G is the gene of interest, encoding the polypeptide of interest.

The fusion partner of choice can be chosen from public domain knowledge, on the basis of previous experience that fusing a gene G to the fusion partner F would improve the expression level and/or the solubility of the entire fusion gene. Examples of fusion partners that have been described for these purposes are:

The gluthation S-transferase gene GST, E. coli thioredoxin TRX, NusA, Chitin binding domain CBD, Chloramphenical acetyl transferase CAT, protein A (protA), LysRS, maltose binding protein (MBP), ubiquitin, calmodulin.

Other fusion partners can be chosen on the basis of rules that predict proteins to be effective as a fusion partner in order to obtain a higher expression and/or to improve the solubility of the fusion construct. US 620,7420 describes such a method based on the hydrophilicity index of the protein identified as a fusion partner.

The fusion partner can also be chosen to include a detection or affinity purification tag into the fusion protein. Of particular interest is a tag that allows for affinity purification of the fusion protein. These tags can be based on chemical principles, or are selected as epitopes for specific antibodies.

Examples of purification tags based on a strong selective chemical interaction to be used as a very selective purification step are a polyhistidine tag (H), that can be used in immobilized metal affinity chromatography, or a polyarginine tag that can be retained in high-salt ion exchange chromatography.

The fusion partner can be chosen from a combination of fusion proteins, a combination of tags or a combination of tags and fusion partners. For example, F could consist of a GST fusion protein F' fused N-terminally to a HIS tag H, where

$$F\text{-}L\text{-}G = H\text{-}F'\text{-}L\text{-}G$$

or, in the case of a combination of fusion partners

$$F1\text{-}F2\text{-}L\text{-}G$$

In such a construct, affinity or detection peptides (tags t) can be placed in and between the fusion proteins or fusion protein domains Fi:

$$(t)\text{-}F1\text{-}(t)\text{-}F2\text{-}(t)\text{-}L\text{-}G$$

The N-terminus of the fusion protein comprising the F-L-G structure can be modified with a signal sequence. A signal sequence is a defined functional unit that allows the fusion protein to be secreted. In eukaryotic cells this will result in delivery of the fusion protein to the endoplasmatic reticulum, and possibly to the extra cellular medium. In Gram positive cells, this signal peptide guides the fusion protein through the cytoplasmic membrane into the medium. In Gram negative- cells the signal sequence guides the fusion protein to the periplasmic space between the cellular and the outer membrane, and occasionally the fusion protein is also released in the culture medium.

In the example where the fusion protein is secreted the formula for the gene construct would be

$$S\text{-}F\text{-}L\text{-}G$$

where S is a signal peptide and F is the fusion partner, there also may consist of a combination of sequences $t_n$ - $F_i$ where n and i are determined according to the application. Other fusion partners or tags can be inserted, either between the linker and the protein of interest or at the C-terminal of the protein of interest. However, these sequences will not be removed from the protein of interest after the linker sequence L is processed by a Caspase enzyme.

The fusion protein comprising the F-L-G parts is made by recombinant DNA techniques. The coding sequence of the complete fusion gene should preferably be determined before cloning. The coding sequences of the different parts in the fusion gene can be assembled using restriction enzymes, which cut specific sites in DNA molecules. Compatible sites can be religated again and this allows for constructing fusion genes. Alternatively, parts of the fusion gene or the complete fusion gene can be constructed by assembling pieces of synthetic DNA. Another possibility is to amplify the regions to be combined by the polymerase chain reaction (PCR) technology, where amplified DNA pieces can be assembled by including appropriate restriction enzymes and/or by including sufficient overlap in the DNA pieces and performing a splice overlap extension PCR reaction. Al of these techniques are known to the person skilled in the art and can be found in recent laboratory manuals covering recombinant DNA technology. Other variations and combinations of these techniques will also allow for obtaining the fusion gene.

## Production of the fusion protein

The fusion gene needs to be engineered in such a way that it is translated into protein by the host organism. As a host organism, any living cell or organism applies. Living cells or organisms can be of prokaryotic or eukaryotic nature. Common cells that serve as hosts for expression of recombinant genes are for instance:

*Escherichia coli, Bacillus species, Streptomyces* species, Yeast strains such as *Saccharomyces, Schizosacharomyces, Pichia* or *Hansenula* strains, Insect cells, mammalian cell lines, plant cells. Expression hosts can also be at the level of a multicellular organism such as transgenic plants, sheep, goat, cow, chicken and rabbit, whereby the product can be isolated either from organs or from body fluids such as milk, blood or eggs.
Alternatively, the gene can be translated into protein using cell free translation systems, possibly coupled to an in vitro transcription system. These systems provide all steps necessary to obtain protein from DNA by supplying the necessary enzymes and substrates in an in vitro reaction. In principle, any living cell or organism can provide the necessary enzymes for this process and extraction protocols for obtaining such enzyme systems are known in the art. Common systems used for in vitro transcription / translation are extracts or lysates from reticolocytes , wheat germ or *Escherichia coli.*

## Isolation of a recombinant fusion protein that can be maturated by caspase

In a preferred embodiment, the fusion polypeptide will be isolated and purified before processing with the caspase protease. In this strategy, the physicochemical features of the fusion part can be used for uniform, streamlined and highly specific purification of the fusion protein. The characteristics of the fusion part towards adsorption chromatographic medium, or specific affinity purification methods should be considered. As non-limiting examples, peptide tags can be included that increase the binding to ion exchange columns (e.g. poly-arginine), hydrophobic interaction columns (e.g. polyphenylalanine), or immobilized metal chelating chromatography (e.g. polyhistidine). Other non-limiting examples are fusion protein or domains that have an affinity for a substrate or ligand (e.g. maltose binding protein MBP, gluthathion S transferase GST, protein A, biotinylated peptides or domains, chitin binding domains CBD). Further non-limiting examples are the use of fusion partners that stay soluble at higher temperature (e.g. thioredoxin), or will reversibly precipitate at certain conditions. A purification scheme based on the properties of the fusion partner will most probably be applicable to the complete fusion protein. A combination of such specific purification methods can be used if the fusion part is composed of different peptides, domains or proteins, or when it shows a different selective behaviour on different chromatography media.

## In vitro cleavage reaction

The in vitro cleavage of a recombinant fusion protein with a caspase should preferably be carried out under specific conditions. It is by no means stated that the invention is limited to these reactions conditions. The pH of the reaction is preferably controlled to be between 6 and 9, more preferably between 7 and 8. There is no need to ad salt to the reaction, but the cleavage reaction can tolerate up to 1M NaCl. Preferably the NaCl concentration is not higher than 0.2 M. Some additives can enhance the cutting reaction of the caspases. Such additives are CHAPS 0.1%, sucrose 10% or mannitol 10%. Caspase enzymes are sensitive to oxidation of the cystein residue in the active centre. In order to avoid this, inclusion of antioxidantia (such as ascorbic acid), reductantia (such as $\beta$-mercaptoethanol, DTT, cysteines) and chelating salts to capture oxidation catalysing ions such as $Zn^{++}$ (such as EDTA).

## On Column cleavage

It is possible to reduce the number of steps needed in the maturation of the fusion protein, subsequent removal of the enzyme and removal of the fusion part cut from the fusion protein. If an affinity tag is incorporated in the fusion part, the same affinity tag can be fused, e.g. by recombinant DNA technology, to the caspase. Using this strategy, the fusion protein can be captured on a solid support (which can be a chromatographic column), and then incubated with a caspase that shows affinity for the same solid support. After an appropriate incubation time, the liquid phase of the reaction vessel will contain the protein of interest, while both the fusion part and the enzyme are adsorbed on the solid phase. Care should be taken to reduce the exposure of the caspase to oxidative conditions, since then the active site cystein will be oxidised and the reaction not reach is full potential. Reductantia (if possible), antioxidantia and chelating salts to capture oxidation catalysts can be included in the reaction. Examples of such conditions are 1-10 mM DTT or β-mercaptoethanol, or 1% ascorbic acid, or 1 mM EDTA.

In vivo cleavage

Cleavage of the fusion peptide can also be induced in vivo. Cleavage in the cell or the medium of the bioreactor has the advantage that no post-productional processing is needed. However, the advantage of a specific affinity purification based on the properties of the fusion part is lost in this case.

Two alternative strategies can be applied. First, the caspase may be induced at the same time as the fusion protein. This can be realized by constructing a transcriptional operon of the caspase enzyme and the fusion protein, or by constructing a translational fusion including the caspase in the fusion protein, or by cloning the caspase behind a separate promoter which is induced at the same time as the one in front of the fusion protein. The latter can be realized by using the same promoter in two transcriptional cassettes, or by using two promoters that are induced with the same inducer (e.g. IPTG/lactose), or by using two promoters, that are inducible with different agents, whereby both agents are added at the same time. Alternatively, the caspase enzyme can be induced at a different time point than the onset of production of the fusion protein. The caspase can be produced before or more preferably after the production onset of the fusion protein. In the latter case, the protein of interest will more likely fold to a soluble, active protein.

A first aspect of the invention is a method of producing a polypeptide of interest in a mature form, comprising

- producing, in a suitable host, a fusion protein that comprises an amino terminal polypeptide, a linker comprising a caspase recognition site and a polypeptide of Interest
- cleaving said fusion protein by a caspase
- isolating the mature polypeptide of interest.

In another aspect the invention relates to a method for producing a protein or polypeptide having a predetermined amino-terminal amino acid residue, comprising: a) expressing the protein or polypeptide in a host cell as a fusion protein wherein the amino terminus of the protein or polypeptide is fused to one or more fusion partners with a linker, the fusion protein being specifically cleavable by a caspase at the junction of the linker with the amino-terminal amino acid residue of the protein or polypeptide, the host cell lacking a caspase which cleaves the fusion protein at the junction of linker and the amino-terminal amino acid residue of the protein or polypeptide; b) isolating the fusion protein from the host cell; and c) contacting the fusion protein with an extract containing a caspase which specifically cleaves the fusion protein at the junction of the linker and the amino-terminal amino add residue of the protein or polypeptide, said extract being derived from cells which produce said caspase by recombinant DNA methods.

The suitable host can be any host, and comprises both eukaryotic and prokaryotic host cells.

As a non limiting example, the host can be a mammalian cell, and Insect cell, a plant cell or a complete plant, a yeast cell, a fungal call, a gram positive bacterium such as *Bacillus* sp or lactic acid bacteria, or a gram negative bacterium such as *E coli*. Preferably said host Is a prokaryotic cell, more preferably said host is a gram-negative bacterium, most preferably said host is *Escherichia coli.*

Preferably, said caspase recognition site comprises the amino acid sequence DELD or DXXD. More preferably, said caspase recognition site comprises either the sequence DEVD or DEHD.

Preferably, said linker sequence has been optimised for optimal caspase processing. Methods to optimise the linker sequence are known to the person skilled in the art, and include, as a non-limiting example, the insertion of a spacer region at the aminoterminal end of the caspase recognition site. The spacer region preferably comprises at least 1, more preferably at least 2, 3, 4, 5, 6, 7, 8, 9, 10, 11, 12, 13 or 14 additional amino acids, more preferably said spacer region comprises at least 15, 20 or 25 additional amino acids.

The caspase used can be any caspase. However, preferably said caspase is selected from the group consisting of caspase-2, caspase-3, caspase-7, CED-3, reversed caspase-3 and reversed caspase-7. More preferably, said caspase is caspase-3 or caspase-7. Even more preferably, said caspase is caspase-3.

In a preferred embodiment, the cleavage by the caspase is carried out in vitro, after isolating the fusion protein from the host cell, by mixing the fusion protein wit a caspase, preferably a purified caspase, in a suitable reaction mixture. However, the caspase cleavage may be carried out in vivo too, by inducing caspase activity in the same host cell as the one where the fusion protein is produced. The induction of the caspase activity may occur simultaneously with the production of the fusion protein, or the production of caspase and fusion protein may be sequential.

The caspase processing is interesting because of its high efficiency, even for mature proteins comprising an amino terminal amino acid that is difficult to process using other proteases. For normal amino terminal amino acids (i.e no P, D or E), preferably a yield of at least 80%, preferably 90% of mature polypeptide of interest is obtained within 60 minutes after initiation of the cleavage reaction. Cleavage of the fusion protein can be judged or examined by eye, ie on an SDS-Page gel, or can be measured by densitometric scanning.

When a mature polypeptide with a proline as amino terminal amino acid is processed, preferably a yield of at least 20%, more preferably a yield of 25% of mature polypeptide is obtained. When a mature polypeptide with either a glutamine or an asparagine as amino terminal amino acid is processed, preferably a yield of at least 60%, more preferably a yield of 75% of mature polypeptide is obtained. Preferably said yields are obtained within 60 minutes after initiation of the cleavage reaction.

Definitions

The following definitions are set forth to illustrate and define the meaning and scope of various terms used to describe the invention herein.

The notation 'DEVD/S' (SEQ ID NO 17) means that a serine (S) follows the DEVD recognition sequence and is released after processing as the first amino acid of the mature protein.

The notation 'DEVD/' means that the polypeptide is cut after the last D irrespective of the first amino acid following the cutting site.

The terms 'protein' and 'polypeptide' as used in this application are interchangeable. 'Polypeptide' refers to a polymer of amino acids and does not refer to a specific length of the molecule. This term also includes post-translational modifications of the polypeptide, such as glycosylation, phosphorylation and acetylation.

The term 'polypeptide of interest' means the polypeptide that should be produced by the processing, and will be used for further applications. Preferably, it is a biological active molecule, such as an enzyme of a cytokine.

The 'mature form' or 'mature polypeptide' of interest refers to the polypeptide of interest in its biologically active form, without prepeptides or leader sequences. Mature form and mature polypeptide is used when we want to stress that the protein is starting with the amino terminal amino acid of the polypeptide of interest occurring under its biological active form. Preferably, said biological active form is identical to the mature form of the polypeptide of interest as it occurs in nature.

The 'yield' of the cleavage reaction is defined as the ratio of the protein of interest that is obtained by the cleavage reaction, on the theoretical amount of protein of interest that can be obtained, assuming that all fusion protein would be cleaved, expressed as a percentage.

**Brief description of figures**

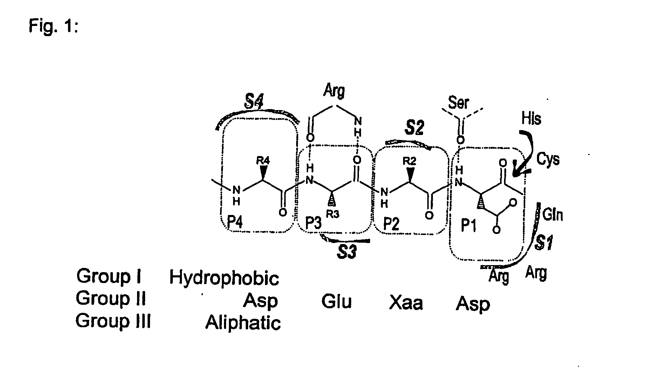

**Figure 1:** Caspase classification towards specificity. The figure shows the target sequence in the active site of the enzyme. The enzyme receiving pockets are denoted S1-S4, while the amino acid positions of the target sequence are denoted P1-P4. While Group II caspases prefers an aspartyl at position P4, Group I caspases prefer a hydrophobic residue at this position and Group III caspases prefer an aliphatic group at the P4 position.

**Figure 2:** Design of a fusion protein
Different linker sequences (1 to 5, corresponding with SEQ ID NO. 1 to 10: even numbers amino acid sequences, uneven numbers nucleic acid sequences) were engineered between the fusion partner and the protein of interest. All constructs were equally expressed as a soluble protein. F: fusion partner, L: linker, P: polypeptide of interest.

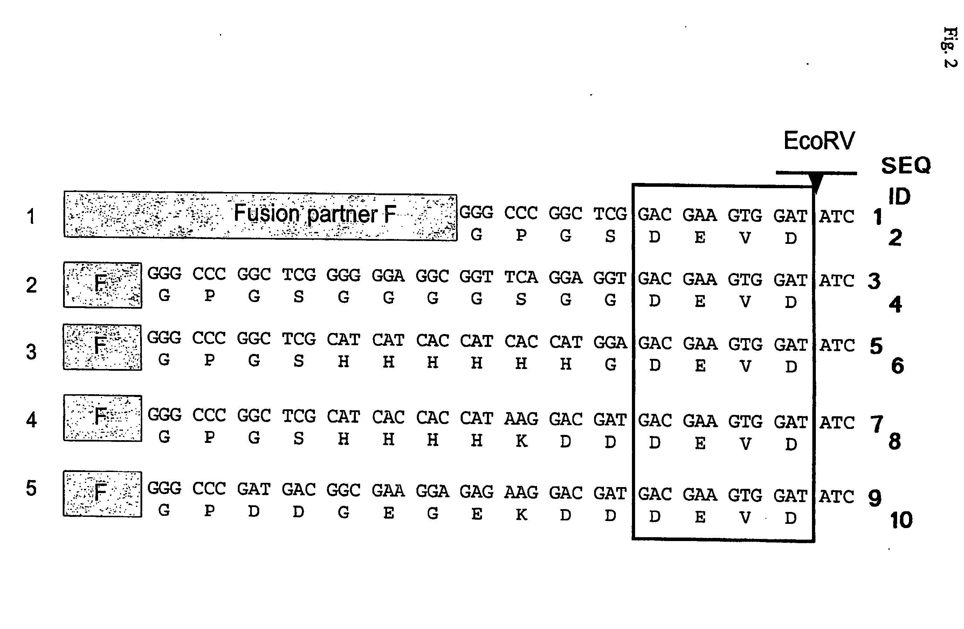

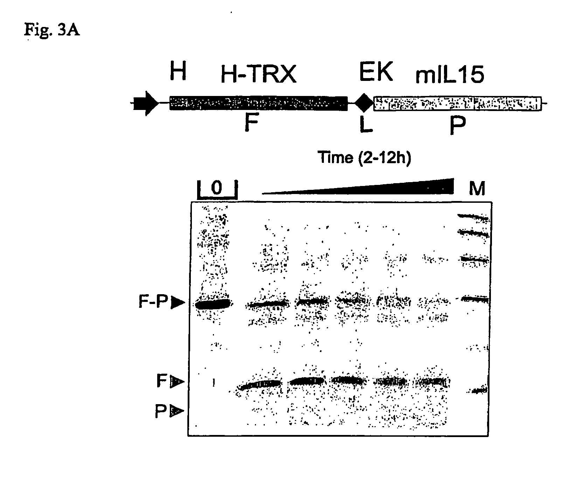

**Figure 3 a) and b):** Enterokinase can either fail to process an engineered linker, or degrade the target protein of interest. Both Caspase-3 and reverse Caspase-7 process the fusion protein with high efficiency in a very short time. EK: enterokinase recognition site; H: histidine tag; GST: glutathion S transferase; TRX: thioredoxin; F,L and P as in figure 2.

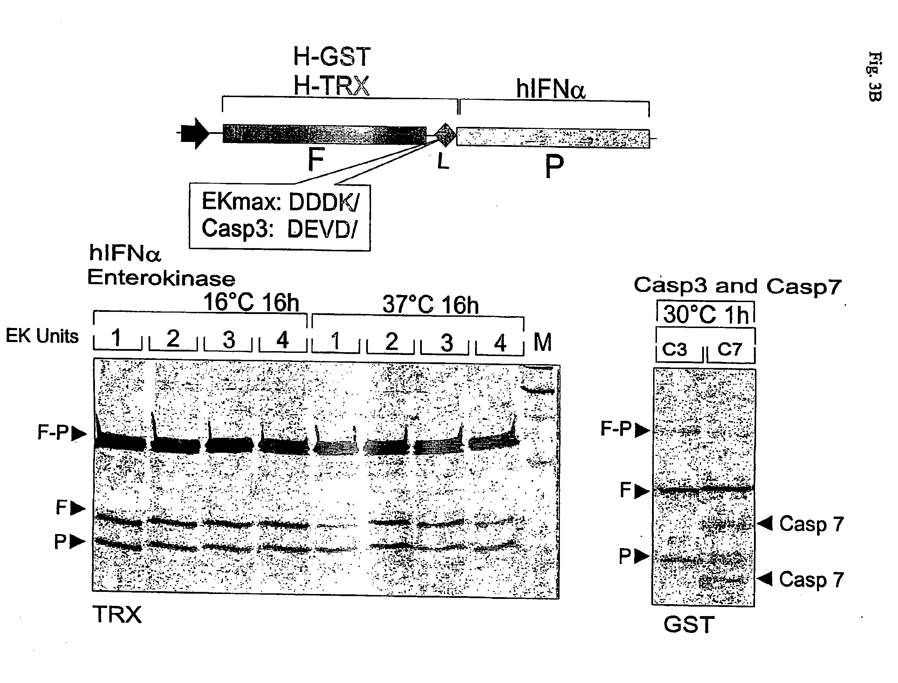

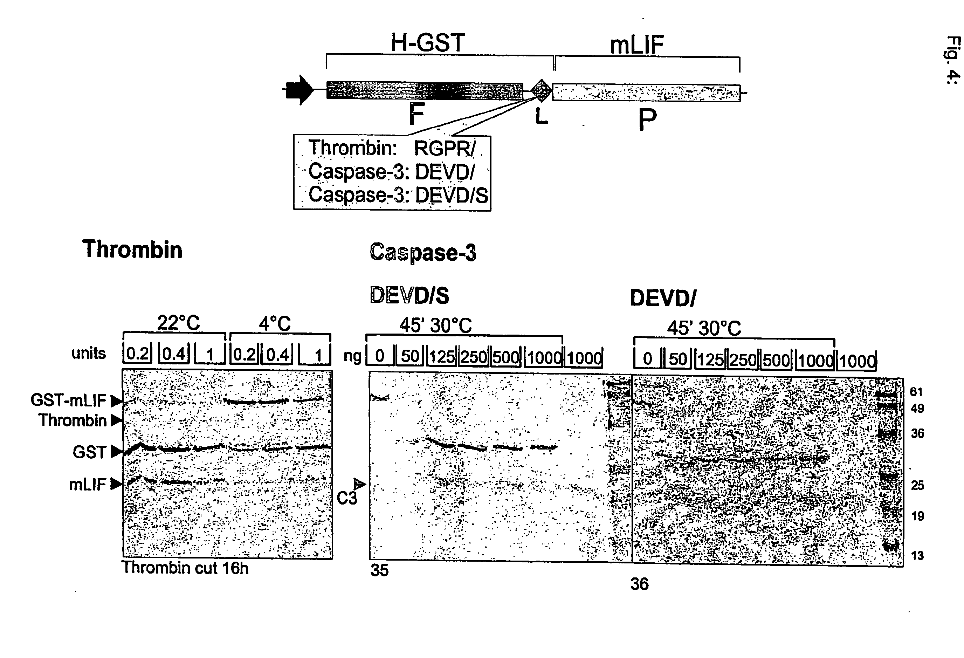

**Figure 4:** Thrombin cutting of a GST-mLIF fusion protein needs a long processing time. This can be done more efficiently and in a very short time with Caspase-3. Two cutting sites were compared (DEVD/ and DEVD/S) who performed equally in the processing reaction. Abbreviations as in figure 3.

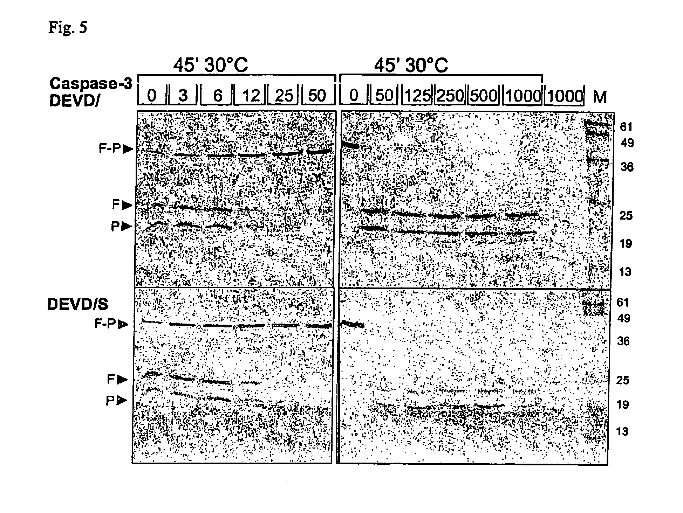

**Figure 5:** The figure shows a titration of enzyme during 16 h incubation for Thrombin, and a 45 minutes incubation for Caspase-3. An amount of 50 ng is sufficient to process >90% of 10 microgram of a GST-mLIF fusion protein.

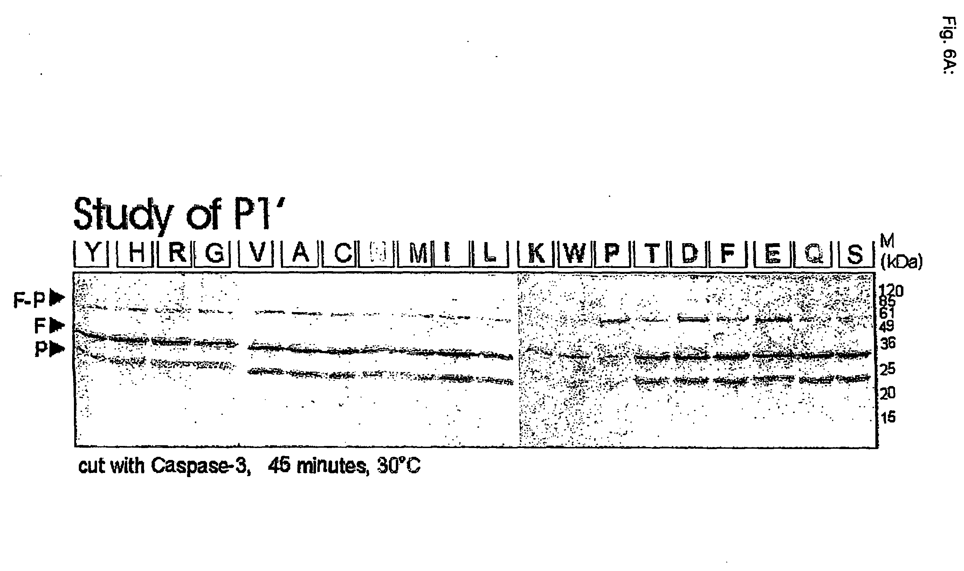

**Figure 6A:** Influence of the P1' site (first amino acid behind, i.e. downstream of, the cutting site) on cutting efficiency. The P1' site was changed to all 20 possible amino acids and assayed for inhibition of processing.

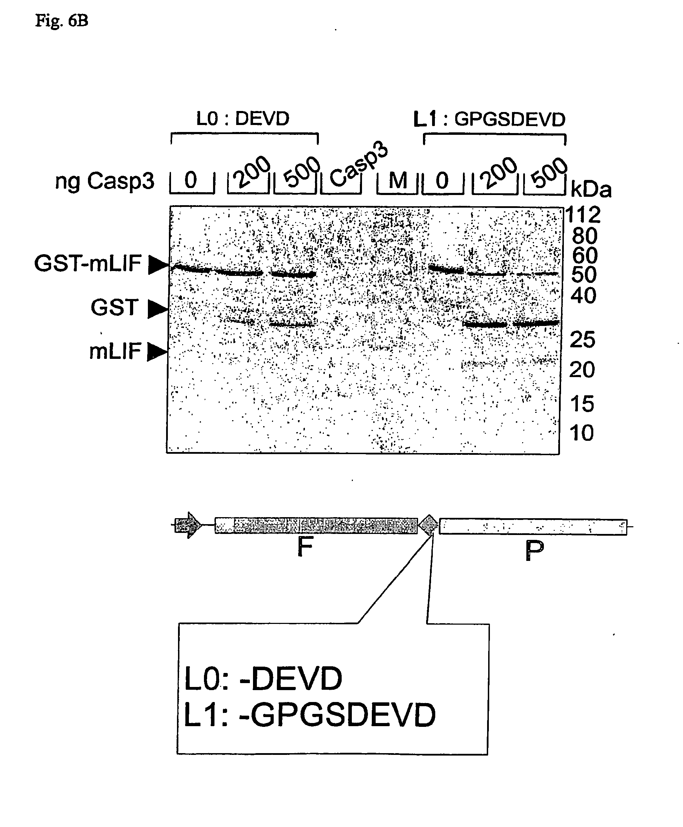

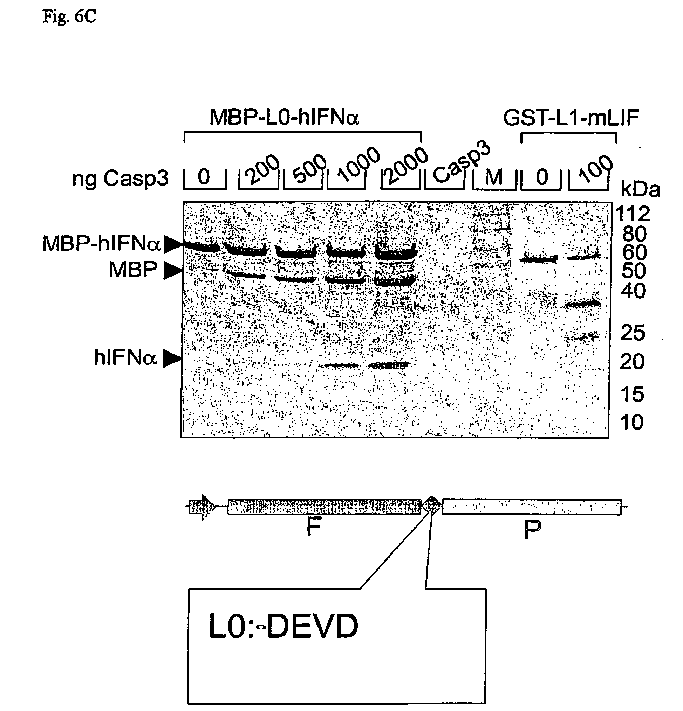

**Figures 6B and 6C:** Influence of the linker sequence on the efficiency of cutting. Linker sequences consisting of a recognition site only are less efficient in cutting the fusion protein. In Figure 6C it is shown that at least 2 $\mu$g of

caspase 3 is needed (left construct) as compared to the control (right construct) where only 100 ng results in efficient cleavage of the fusion protein.

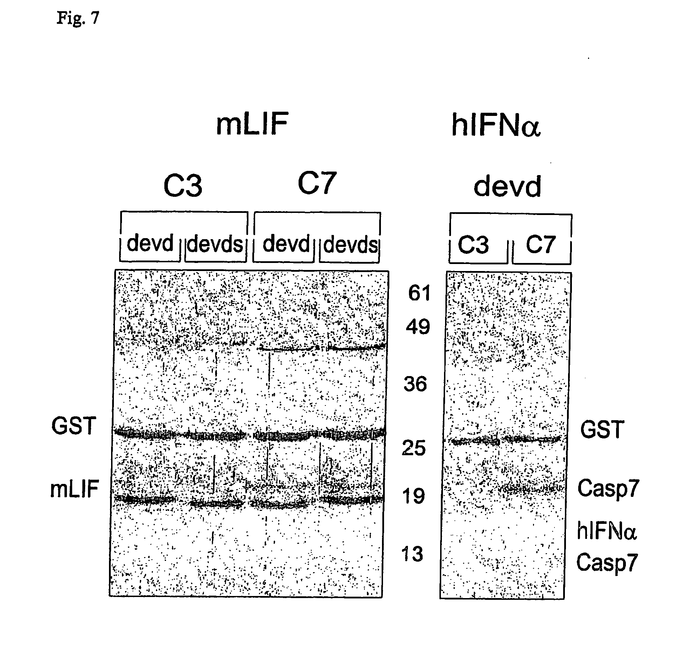

**Figure 7:** Both mLIF and hIFN alpha were cut efficiently using Either Caspase-3 or reverse Caspase-7, with either the recognition site DEVD/ or DEVD/S.

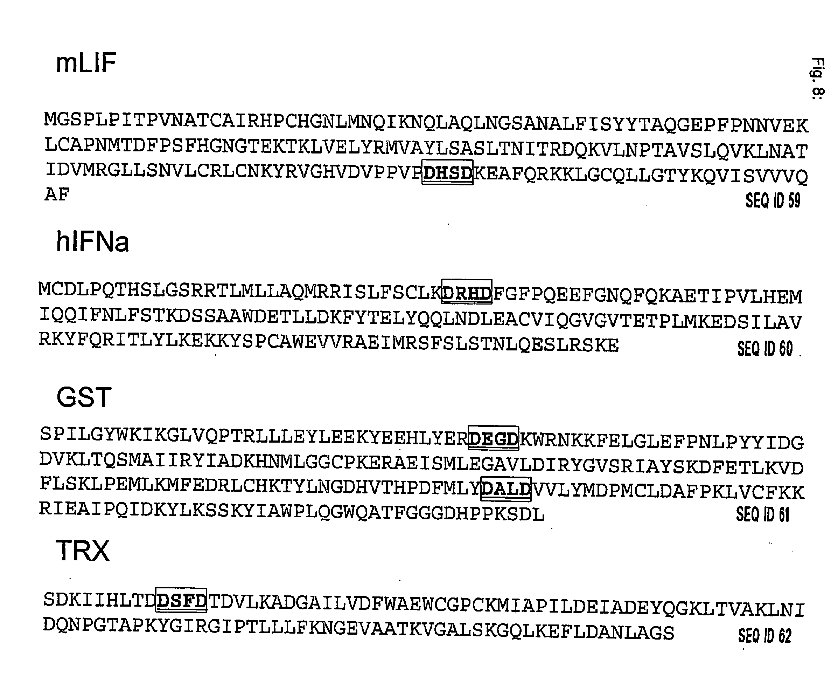

**Figure 8**: Screening of the amino acid sequence of mLIF, hIFN alpha, GST and TRX. Consensus sequences for Caspase group II recognition sites (DXXD) are boxed.

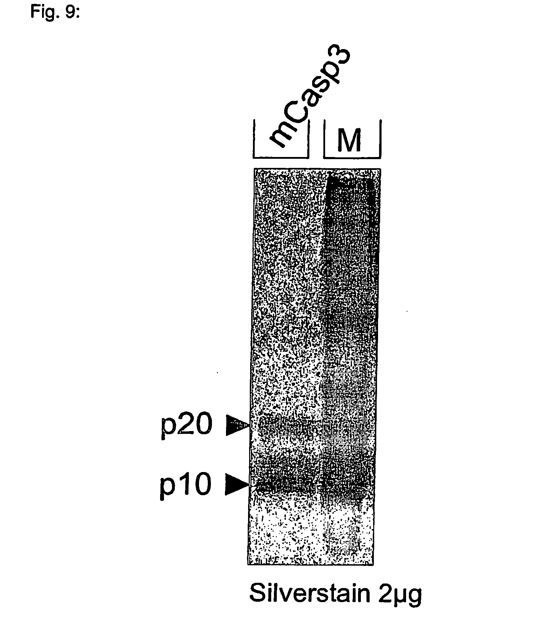

**Figure 9:** Expression and purification of Caspase enzymes. Silver stain of Caspase-3 produced in Escherichia coli and purified as described.

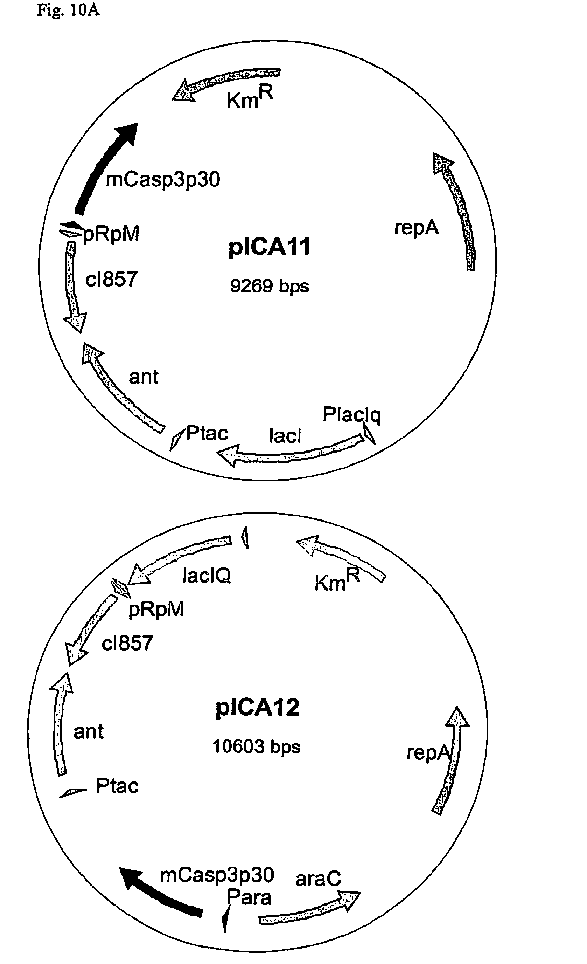

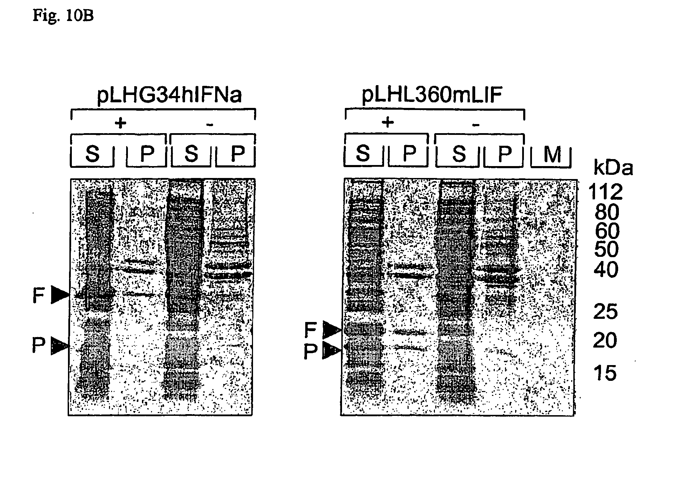

**Figure 10:** Co-expression of caspase with a fusion protein allows cleavage in vivo. A) plasmid maps for simultaneous or separate induction of caspase 3. B) Protein gel analysis of bacteria co-expressing caspase 3 with a cleavable fusion protein.

## Examples

### Example 1 Designing a cleavable linker sequence for caspases maturation

In order to use the method of the invention, one should first design an expression vector for a fusion protein construct, where the fusion construct is separated from the mature protein by a linker sequence. The linker sequence must contain a preferred recognition sequence for a Caspase protein. Examples of such recognition sequences are given in Table I. Figure 2 shows some examples of linkers that were used for cleavage with Caspase 3.

### Example 2 Cleavage with Caspase 3 outperforms cleavage with industry standard proteases, which lack efficiency or specificity

Industry standard enzymes for processing fusion proteins in order to obtain a mature protein without any additional amino acid, residual from designing the cleavage site, are not always efficient and can constitute the bottleneck in designing an efficient production process. Figure 3 a) shows a cleavage experiment of a thioredoxin-murine interleukin 15 fusion gene, separated from each other with a recognition site for enterokinase (TRX-EK-mIL15). Cleavage with enterokinase required a long incubation time in order to process the molecule. In the end, the molecule was processed, which is apparent by the release of the thioredoxin (TRX) protein, but the murine interleukin 15 protein (mIL15) is also degraded by the enzyme or the enzyme preparation. In the other example, in Figure 3 b), a fusion protein of TRX with human interferon alpha (hIFNa) comprising a linker sequence encoding a recognition site for Enterokinase (DDDK), TRX-EK-hIFNa, was compared to a fusion construct of gluthathion-S-tranferase (GST) with hIFNα comprising a Caspase-3 recognition site (DEVD) in the linker sequence, GST-C-hIFNα in the linker sequence. The fusion proteins were produced, purified, and processed with respectively Enterokinase and Caspase-3. 16h incubation with Enterokinase resulted in only a minor amount of processing of the fusion protein. Caspase-3 on the other hand gave a high yield of processing even after only 45 minutes of incubation. Another industry standard for a protease to trim fusion proteins to obtain an unmodified mature protein sequence is Thrombin. Figure 4 shows an example where murine LIF is fused to a GST fusion partner and a hexahistidine fusion partner (H), comprising either a thrombin protease recognition sequence in the connecting linker (H-GST-T-mLIF), or a Caspase-3 recognition sequence (H-GST-C-mLIF). Two different sites for Caspase-3 were introduced. Since it can be concluded from the analysis of the natural Caspase-3 sites that there is a clear preference for a small amino acid at the P1' position, a linker sequence comprising a DEVD/ recognition sequence where the mature mLIF sequence follows the sequence and is released after processing, was compared with a linker sequence comprising a DEVD/S, where a serine-mLIF follows the DEVD recognition sequence and is released after processing. The processing with Caspase-3 was far more efficient as compared with Thrombin, and the reaction was finished after 45 minutes when using Caspase-3 while up to 16 h were needed to process the H-GST-T-mLIF fusion protein with Thrombin. To our surprise, we could not see any difference in processing efficiency when using the H-GST-DEVD/mLIF or H-GST-DEVD/S-mLIF fusion proteins.

To test the efficiency of cutting of such an engineered fusion protein, we titrated the enzyme on fusion protein. Figure 5 shows an example of such a titration experiment on a H-GST-DEVD/mLIF and a H-GST-DEVD/S-mLIF. Again, no difference could be seen between both constructs, and the titration reaction shows that as little as 50 ng of enzyme is sufficient to process 10 microgram of protein.

To test the limits of the Caspase-3 processing and the limitations in processing fusion proteins imposed by the P1' sequence, we inserted 20 different amino acid at the P1' position, preceding the mLIF sequence. All the 20 fusion proteins of the formula H-GST DEVD/X-mLIF, where X is any of the possible 20 amino acids described by their single letter code, where expressed in E. coli, purified and used as substrate for cleavage with Caspase-3. Figure 6 shows the results of the cutting experiment. To our great surprise, all amino acids in the P1' position could be processed by Caspase-3. Proline (P) at position P1' inhibited the processing about 75%, and the acidic amino acids glutamine (E) and asparagine (D) inhibited the processing about 25%, but no amino acid inhibited the Caspase processing 100%.

Next, we assessed the influence of the preceding amino acids by shortening and varying the linker sequences. Different lengths and sequences of the linker sequences before the DEVD-site had no influence on the cleavage reaction (figure 2). However, when no preceding linker sequence was present and the DEVD site followed the fusion protein directly, a marked decrease in the efficiency of cutting was noticed. Figure 6B shows the fusion protein of GST and mLIF but now with only the DEVD site in the linker (L0: DEVD) compared to the construct where the DEVD site is preceded by a GPGS sequence (L1: GPGSDEVD). While 90% of the fusion product containing L1 was processed (as measured by densitometric scanning of the gel), only 10% processing yield was seen under these conditions when using the L0 linker.

Figure 6C shows a second example where 2 microgram of caspase 3 is needed to process only 50% of 10 microgram of MBP-LO-hIFNalpha. For 10 microgram of the control construct only 100 ng of caspase 3 results in efficient cleavage.

Figure 7 shows that essentially the same results can be obtained by using caspase 7 or reversed caspase 7. In our hands, the murine caspase 7 was not as active as the murine caspase 3 and more enzyme needed to be added to obtain the same efficiency of cleavage.

The specificity of the cutting reaction is illustrated by analysis of the sequence of the target proteins used in the example: both mLIF and hIFNa contain a consensus target sequence for group II caspases (DXXD/). Also the fusion partners used in these experiments contain the DXXD consensus sequence for group II caspase. Thioredoxin (TRX) contains a site, while the GST fusion partner even contains 2 target sequences (figure 8). One would expect further degradation of a fusion product comprising these proteins. To our great surprise, none of these sites was cut even upon prolonged incubation with Caspase-3 or Caspase-7, proving the specificity of the reaction towards the engineered sequence in the linker.

We conclude that the Caspases are a good choice to be used as a processing enzyme for fusion proteins since any amino acid can be tolerated at the P1' position, they are efficient in cutting (usually complete after 45 minutes reaction time), and the sequence specificity is sufficient to prefer the processing at the engineered site, even if the target protein contains consensus recognition sequences for the Caspase enzymes.

## Example 3 Expression and purification of caspases

The primary structure of the caspase zymogens or procaspases consists of a prodomain followed by a large subdomain of around 20 kDa (p20), and a smaller subdomain of around 10 kDa (p10). A combination of the p10 and p20 is denoted as p30. Active caspase can be obtained by co-expressing the processed subunits as separate subunits. In eukaryotic expression hosts this can be done by co-transfection of two plasmids containing both a promoter followed by a p10 or a p20 encoding gene. Alternatively, an internal ribosomal entry site can be used to express both subunits from a single transcript. In prokaryotes, also two promoter construct can be made, or the two genes can be placed in a single operon. The correct start position of both coding sequences should be engineered to optimize translation initiation in the host cell chosen, as known in the art.

Also, the caspase enzyme can be expressed as a p30, or as a procaspase. For most caspases, overexpression will induce self-activation by autoprocessing, resulting in the release of the p10 and the p20 from the p30 or the procaspase in vivo. The p10 and the p20 form a heterodimer that in turn will dimerise, so the final enzyme is a (p10-p20)2 tetramer. Full length cDNA of caspase genes was used to clone the p30 caspases. Using synthetic oligonucleotide primers, appropriate restriction sites were engineered for subsequent cloning. The p30 caspase cDNA was clones behind the lambda PL promoter as described in Van de Craen et al. 1999, Cell Death and Differentiation a, 1117-1124, and induced by induction of an antirepressor as described in WO9848025. For convenience, the Caspase enzymes were tagged with a hexahistidine sequence at the N-terminus of the p20 sequence, or at the C-terminus of the p10 sequence, if the caspase is in the natural orientation. A reversed orientation can also be engineered as described in US 26379950. In this case, the polyhistidine tag was either at the N-terminus of the p10, or at the C-terminus of the 20.

Upon induction of the Caspase gene in Escherichia coli according to WO9848025, the processed forms of the p10 and of the p20 were obtained. The bacterial pellet was resuspended in buffer A containing 20 mM Tris HCl pH 7.5; 10% glycerol; 1 mM oxidized gluthathione, 500 mM NaCl, 1 mM phenylmethylsulfonyl fluoride (PMSF), 50 mM leupeptine and 20 µg/ml aprotinin. The resuspended pellet was lysed by sonication or in a French Press. Insoluble proteins were removed by centrifugation or ultrafiltration. The cleared lysate of the bacterial culture was passed over a DEAE column to remove bacterial DNA. The flow-through was loaded on an immobilized metal chelating column containing 50 mM imidazol. Bound material was eluted from the column using 200 mM imidazol with 20 mM Tris-HCl pH 7.5, 10% glycerol, 1 mM oxidised gluthathione, 50 mM NaCl and dialysed to PBS buffer containing 1 mM oxidised gluthathione. The sample was applied over a mono-Q column (Amersham Bioscience) and eluted with a gradient of 0 to 1 M NaCl. Figure 9 show a typical preparation of purified murine Caspase-3 from Escherichia coli.

Expression of the caspases could be increased by using fusion proteins. The caspases are fused to the C-terminus of a fusion partner such as thioredoxin, gluthathione S-transferase, maltose binding protein, NusA or others. The fusion between the fusion partner and the caspase gene can contain a protease site to release the caspase after production. Also, this protease site can be a target site for the Caspase itself, so the fusion protein is autoprocessed upon production of the active caspase.

## Example 4 : Caspases maturation of fusion proteins in vivo

The cleavage reaction can be induced in vivo by cotransfecting the host cell with an expression vector for the Caspase enzyme. We have illustrated this by cloning the caspase gene behind the lambda PR promoter present on pICA2 plasmid that is described in WO9848025. A derivative of pICA2 had the promoter controlling ant (PN25/02) changed to the Ptac promoter (pICA10). Next the gene for murine caspase 3 was cloned behind the PR promoter. The resulting pICA11 vector induces caspase together with the fusion protein (figure 10A). This results in the cleavage of a fusion protein comprising a DEVD in the linker sequence. Figure 10B illustrates this using a GST-L1-IFNalpha fusion and a cleavable fusion protein containing mLIF. Quantitative cleavage occurred in vivo and no unprocessed fusion protein F-L1-P could be detected. Since in some cases the protein of interest needs a certain time to fold correctly, even when fused to the fusion partner, it is preferable that the induction of the Caspase enzyme will be independently regulated from the induction of the fusion protein. In this strategy, it is most preferable to first induce the fusion protein and after a time to be determined experimentally, induce the Caspase enzyme. Since the Caspase enzymes are active in the cytoplasm of E. coli and other host organisms, the fusion protein will be cut in vivo and the mature protein will be released. This strategy looses the advantage of a possibility for affinity purification, but avoids the process of in vitro cleavage and the consumption of purified Caspase enzyme.

A Caspase-3 p30 protein was cloned fused to a hexahistidine tag behind a Xanthosine promoter as described in PT102705. The H-GST-DEVD/mLIF fusion protein was induced using the lambda PUant induction system as described in WO9848025. After 4 h of induction by the addition of IPTG 1 mM, 0.2% of xanthosine was added to the medium. The Caspase-3 activity is induced and processes the fusion protein in vivo. In another strategy, the caspase 3 gene was cloned behind the arabinose promoter and inserted in pICA10. The resulting pICA12 (figure 10A) induces caspase upon the addition of 0.1% arabinose, while the fusion protein will be induced by the addition of lactose or IPTG.

## Example 5: A caspases maturation bioreactor

In order to take advantage of the possibility to use the fusion construct for affinity purification, but to minimize the consumption of purified enzyme, a bioprocess reactor can be devised based on Caspase enzyme coupled to a solid support. In this way, easy recuperation can be achieved and the enzyme reactor can be reused several times. Also, the removal of the enzyme is not necessary anymore since the enzyme remains bound to the solid phase support and thus does not contaminate the protein of interest, which is in the liquid phase. Caspase- was purified and coupled to a preactivated NHS column (Amersham Bioscience) according the supplier's instructions. Non-bound enzyme was removed by several washes. A purified fraction of GST-L-mLIF was loaded into the reactor, and incubated for 2 hours. The reactor was flushed and the eluted protein captured on an immobilized metal affinity column. The flow-through contains the processed protein of interest while the fusion part is captured on the IMAC column. The Caspase-3 reactor could be used more than once.

## Table I

All proteins are referenced in Earnshaw, Martins and Kaufmann, Mammalian caspases: structure, activation, substrates and functions during apoptosis. Annu. Rev. Biochem; 1999, 68:383-424.

| Naturally occurring target sites for Group II caspases (2, 3, 7) | | | | | | | | |
|---|---|---|---|---|---|---|---|---|
| Consensus | | | Lacking P3 Glu | | | Lacking P4 Asp | | |
| Protein | P4-P1 | P1' | Protein | P4-P1 | P1' | Protein | P4-P1 | P1' |
| PARP | DEVD | G | MEKK-1 | DTVD | G | Stat1 | MELD (40*) | A |
| RepC LS | DEVD | G | FAK (a) | DQTD (25*) | S | Cytokeratin 18 | VEVD (41*) | A |
| BetaII-spectrin | DEVD | S | PKSrelK2 | DITD (26*) | C | Lamin B1 | VEVD | S |
| Fodrin (a) | DETD (18*) | S | PKCdelta | DMQD (27*) | M | FLIPL | LEVD (42*) | G |
| Mst1 K | DEMD (19) | S | Fodrin (a) | DSLD (28*) | S | Lacking P3 Glu + P4 Asp | | |
| Mst2 K | DELD (20*) | S | 70kDaU1snRNP | DGPD (29*) | G | Bid | LQTD (43*) | G |
| D4 GDP DI | DELD | S | mdm2 | DVPD (30*) | C | FAK (a) | VSWD (44*) | S |
| SREBP | DEPD (21*) | S | IkappaB | DRHD (31*) | A | PITSLRE 2-1 | YVPD (45*) | S |
| Rb | DEAD (22*) | G | Waf1 | DHVD (32*) | L | p21 K2 | SHVD (46*) | G |
| PP2A Aalpha | DEQD (23*) | S | Kip1 | DPSD (33*) | S | Bcl-X | HLAD (47*) | S |
| ICAD | DEPD | S | Huntington | DSVD (34*) | L | Presenilin-1 | ARQD (48*) | S |
| PPLA2 | DELD | A | DPAP | DSLD (35*) | S | Ca PK IV | PAPD (49*) | A |
| | | | Presenilin-2 | DSYD (36*) | S | pro-IL-6 | SSTD (50*) | S |
| Larger P1' | | | Gelsolin | DQTD (37*) | G | PKN | LGTD (51*) | S |
| DNA-P-Kos | DEVD (24*) | N | TopoI | DDVD (38*) | Y | DCC | LSVD (52*) | R |
| PKCteta | DEVD | K | Bcl-2 | DAGD (39*) | V | Calpastatin (a) | ALDD (53*) | S |
| | | | RAS-GAP | DTVD | G | Calpastatin (b) | LSSD (54*) | F |
| | | | | | | Calpastatin (c) | ALAD (55*) | S |
| | | | | | | Bax | FIQD (56*) | R |

* The numbers in brackets refer to the corresponding SEQ ID NOs.

## References

Colussi, P. A., N. L. Harvey, L. M. Shearwin-Whyatt and S. Kumar (1998). "Conversion of procaspase-3 to an autoactivating caspase by fusion to the caspase-2 prodomain." J Biol Chem 273(41): 26566-70.

Garcia-Calvo, M., E. P. Peterson, D. M. Rasper, J. P. Vaillancourt, R. Zamboni, D. W. Nicholson and N. A. Thornberry (1999). "Purification and catalytic properties of human caspase family members." Cell Death Differ 6(4): 362-9.

Nicholson, D. W. (1999). "Caspase structure, proteolytic substrates, and function during apoptotic cell death." Cell Death Differ 6(11): 1028-42.

Nicholson, D. W. and N. A. Thornberry (1997). "Caspases: killer proteases." Trends Biochem Sci 22(8): 299-306.

Stennicke, H. R., M. Renatus, M. Meldal and G. S. Salvesen (2000). "Internally quenched fluorescent peptide substrates disclose the subsite preferences of human caspases 1, 3, 6, 7 and 8." Biochem J 350 Pt 2: 563-8.

Stennicke, H. R. and G. S. Salvesen (1997). "Biochemical characteristics of caspases-3, -6, - 7, and -8." J Biol Chem 272(41): 25719-23.

Stennicke, H. R. and G. S. Salvesen (1999). "Catalytic properties of the caspases." Cell Death Differ 6(11): 1054-9.

Thornberry, N. A., K. T. Chapman and D. W. Nicholson (2000). "Determination of caspase specificities using a peptide combinatorial library." Methods Enzymol 322:100-10.

Van de Craen, M., W. Declercq, I. Van den brande, W. Fiers and P. Vandenabeele (1999). "The proteolytic procaspase activation network: an in vitro analysis." Cell Death Differ 6(11): 1117-24.

Alnemri, E. (1999). Recombinant, active caspases and uses thereof. US6379950.

McCoy, J. and E. Lavallie (1993). Peptide and protein fusions to thioredoxin and thioredoxin-like molecules. WO9213955.

Smith, D. (1993). Tripartide fusion proteins of gluthathione S-transferase. WO9319091

Smith, D. (1988). Novel fusion proteins containing gluthathione S-transferase. WO8809372

LaVallie, E. (1994). Cloning of Enterokinase and method of use. US5665566.

Johnston, S. and W. Dougherty (1993). Method of isolation and purification of fusion polypeptides. US5532142

**Claims**

1. A method for producing a polypeptide of interest in a biologically active form, comprising

   - producing, in a suitable host, a fusion protein comprising an aminoterminal polypeptide, a linker sequence comprising a caspase group II recognition site and the polypeptide of interest,
   - cleaving said fusion protein by a caspase suitable for recognizing said caspase group II recognition site, which specifically cleaves the fusion protein at the junction of the linker sequence and the amino-terminal amino acid residue of the mature polypeptide of interest, and
   - isolating the biologically active polypeptide of interest.

2. The method according to claim 1 wherein said suitable host is a prokaryote

3. The method according to claim 2 wherein said prokaryote is *Escherichia coli*

4. The method according to any of claims 1 to 3 wherein said caspase recognition site comprises the amino acid sequence DELD, DXXD, DEVD or DEHD.

5. The method according to any of claims 1 to 4 wherein said caspase is selected from the group consisting of caspase-2, caspase-3, caspase-7, CED-3, reversed caspase-3 and reversed caspase-7.

6. The method according to any of claims 1 to 5 wherein said linker sequence comprises at least 5 amino acids.

7. The method according to any of claims 1 to 6 wherein said linker sequence is represented by any of SEQ ID NOs 2, 4, 6, 8, 10, 13, 14, 20 or 63.

8. The method according to any of claims 1 to 7 wherein said cleaving of the fusion protein by the caspase is carried out in vitro.

9. The method according to any of claims 1 to 8 wherein a yield of at least 80% of mature polypeptide of interest is obtained within 60 minutes as judged by densiometric scanning.

10. The method according to any of claims 1 to 7 further **characterized in that** the caspase protein is produced in the same host as the fusion protein.

11. The method according to any of claims 1 to 7 further **characterized in that** the caspase protein sequence is comprised in the fusion protein.

12. The method according to claim 10 or 11, further **characterized in that** the production of the caspase protein and the fusion protein is sequential.

13. The method according to claim 12 **characterized in that** the caspase protein is produced after the fusion protein.

14. The method according to any of claims 9 to 13 wherein said cleaving of the fusion protein is carried out in vivo.

15. A method for producing a polypeptide of interest in a biologically active form, comprising:

   - producing in E. coli , a fusion protein comprising an aminoterminal polypeptide, a linker sequence comprising a caspase group II recognition site and the polypeptide of interest, wherein the linker sequence is represented by any of SEQ ID NOs 2.4. 6. 8.10,13. 14. 20 or 63.
   - cleaving said fusion protein by a caspase suitable for recognizing said caspase group II recognition site, which specifically cleaves the fusion protein at the junction of the linker sequence and the amino-terminal amino acid residue of the mature polypeptide of interest, and
   - isolating the biologically active polypeptide of interest.

16. A method for producing a polypeptide of interest in a biologically active form, comprising:

   - producing in E. coli, (1) a fusion protein comprising an aminoterminal polypeptide, a linker sequence comprising a caspase group II recognition site, the polypeptide of interest and a (2) a caspase protein, suitable for recognizing said caspase group II recognition site, wherein said fusion protein and said caspase polypeptide are under the control of a same or a different promoter,
   - inducing expression of the fusion protein,
   - inducing expression of the caspase protein,
   - cleaving said fusion protein in vivo by the caspase protein which cleaves the fusion protein at the junction of the linker sequence and the amino-terminal amino add residue of the mature polypeptide of interest, and
   - isolating the biologically active polypeptide of interest.

17. The method according to claim 16 wherein the amino acid sequence of the linker is represented by any of SEQ ID NOs 2, 4, 6, 8, 10, 13, 14, 20 or 63.

18. The method according to any of claims 15 to 17 wherein said caspase protein is caspase-3 or caspase-7.

19. The method according to any of claims 16 to 18 wherein a single polynucleic acid encodes the fusion protein and the caspase.

20. The method according to any of claims 16 to 18 wherein separate polynucleic acids encode the fusion protein and the caspase.

21. A polynucleic add encoding a caspase protein and encoding a fusion protein comprising an aminoterminal polypeptide, a mature polypeptide of interest and a linker sequence encoding a peptide represented in any of SEQ ID NOs 2, 4, 6, 8, 10 between the aminioterminal polypeptide and the mature polypeptide of interest, said linker sequence comprising a caspase group II recognition site at the junction between the linker and the amino terminal amino acid of the mature polypeptide of interest.

22. A polynucleic acid according to claim 21 wherein said caspase protein is selected from the group consisting of caspase-2, caspase-3, caspase-7, CED-3, reversed caspase-3 and reversed caspase-7.

23. A polynucleic acid according to claims 21 or 22 wherein said aminoterminal polypeptide is selected from the group consisting of glutathione S-transferase gene GST, E. coli thioredoxin TRX, NusA, chitin binding domain CBD, chloramphenicol acetyl transferase CAT, protein A (prot A), LysRS, maltose binding protein (MBP), ubiquitin, calmodulin, DsbA, DsbC and lambda gpV.

24. A polynucleic acid according to any of claims 21 to 23 wherein said linker sequence comprises at least 5 amino acids.

25. A polynucleic acid according to any of claims 21 to 24 wherein said caspase recognition site consists of the amino acid sequence DELD, DXXD, DEVD or DEHD.

26. A polynucleic acid according to any of claims 21 to 25 wherein the expression of said fusion protein and/or said caspase protein is under the control of a suitable promoter.

27. A polynucleic acid according to claim 26 wherein the fusion protein and the caspase protein are under the control of a same or a different promoter.

28. A polynucleic acid according to claim 26 or 27 wherein said promoter is an inducible promoter.

29. A polynucleic acid according to any of claims 21 to 28 wherein said fusion protein further comprises a detection or affinity tag.

30. A polynucleic acid according to claim 29 wherein said detection or affinity tag is selected from the group consisting of a polyhistidine tag, a polyarginine tag, a streptavidin binding tag, a biotinylated tag and a tag recognized by an antibody.

31. A vector comprising the polynucleic acid of any of claims 21 to 30.

32. A host cell comprising the polynucleic add of any of claims 21 to 30 or comprising the vector of claim 31.

33. A fusion protein encoded by the nucleic acid of any of claims 21 to 30.

34. Use of a polynucleic acid according to any of claims 21 to 30, a vector of claim 31 or a host cell according to claim 32 for the production of a polypeptide of interest in a mature and/or biologically active form.

35. A bioreactor suitable for the production of mature proteins of interest comprising :

  (a) a fusion protein encoded by a polynucleic acid of claim 29, said fusion protein comprising an affinity tag having a specific affinity for a substrate, and
  (b) a substrate,

wherein the affinity tag portion of the fusion protein is bound to the substrate.

36. A bioreactor suitable for the production of mature proteins of interest comprising:

(a) a fusion protein encoded by a polynucleic acid of claim 29, said fusion protein comprising an affinity tag having a specific affinity for a substrate, and

(b) a caspase enzyme coupled to or with affinity for a solid support,

wherein the caspase enzyme specifically cleaves the caspase recognition site in the fusion protein.

37. A method for producing a protein of interest comprising:

(a) producing a fusion protein according to claim 29, said fusion protein comprising an affinity tag or an aminoterminal polypeptide having a specific affinity for a substrate,

(b) introducing said fusion protein into a bioreactor comprising a caspase enzyme coupled to a solid support.

(c) incubating said fusion protein with the caspase enzyme during an appropriate incubation time, and

(d) washing the reactor and capturing the eluted fusion protein by binding to the substrate.

**Patentansprüche**

1. Ein Verfahren zur Herstellung eines Polypeptides von Interesse in einer biologisch aktiven Form, umfassend:

- Herstellung eines Fusionsproteins, umfassend ein aminoterminales Polypeptid, eine Linkersequenz umfassend eine Erkennungsstelle für eine Caspase Gruppe II und den Polypeptides von Interesse, in einem geeigneten Wirt,

- Spaltung des Fusionsproteins durch eine geeignete Caspase für die Erkennung der Erkennungsstelle der Caspase Gruppe II, die spezifisch das Fusionsprotein an der Verbindungsstelle der Linkersequenz und dem aminoterminalen Aminosäurerest des reifen Polypeptides von Interesse spaltet, und

- Isolierung des biologisch aktiven Polypeptides von Interesse.

2. Das Verfahren nach Anspruch 1, wobei der geeignete Wirt ein Prokaryot ist.

3. Das Verfahren nach Anspruch 2, wobei der Prokaryot *Escherichia coli* ist.

4. Das Verfahren nach einem der Ansprüche 1 bis 3, wobei die Caspase-Erkennungsstelle die Aminosäuresequenz DELD, DXXD, DEVD oder DEHD umfasst.

5. Das Verfahren nach einem der Ansprüche 1 bis 4, wobei die Caspase aus der Gruppe, bestehend aus Caspase-2, Caspase-3, Caspase-7, CED-3, reverse Caspase-3 oder reverse Caspase-7, ausgewählt ist.

6. Das Verfahren nach einem der Ansprüche 1 bis 5, wobei die Linkersequenz mindestens 5 Aminosäuren umfasst.

7. Das Verfahren nach einem der Ansprüche 1 bis 6, wobei die Linkersequenz einer der Sequenzen SEQ ID NOs 2, 4, 6, 8, 10, 13, 14, 20 oder 63 entspricht.

8. Das Verfahren nach einem der Ansprüche 1 bis 7, wobei die Spaltung des Fusionsproteins durch die Caspase in vitro erfolgt.

9. Das Verfahren nach einem der Ansprüche 1 bis 8, wobei eine Ausbeute, ermittelt durch densiometrische Bestimmung, von mindestens 80% des reifen Polypeptides von Interesse innerhalb von 60 Minuten erreicht wird.

10. Das Verfahren nach einem der Ansprüche 1 bis 7, **dadurch gekennzeichnet, dass** das Caspase-Protein im gleichen Wirt hergestellt wird wie das Fusionsprotein.

11. Das Verfahren nach einem der Ansprüche 1 bis 7, **dadurch gekennzeichnet, dass** das Fusionsprotein die Sequenz des Caspase-Proteins umfasst.

12. Das Verfahren nach Anspruch 10 oder 11, **dadurch gekennzeichnet, dass** die Herstellung des Caspase-Proteins und des Fusionsproteins sequentiell verläuft.

13. Das Verfahren nach Anspruch 12, **dadurch gekennzeichnet, dass** das Caspase Protein nach dem Fusionsprotein hergestellt wird.

**14.** Das Verfahren nach einem der Ansprüche 9 bis 13, wobei die Spaltung des Fusionsproteins in vivo erfolgt.

**15.** Ein Verfahren zur Herstellung eines Polypeptides von Interesse in einer biologisch aktiven Form, umfassend:

- Herstellung eines Fusionsproteins, umfassend ein aminoterminales Polypeptid, eine Linkersequenz umfassend eine Erkennungsstelle für eine Caspase Gruppe II und den Polypeptid von Interesse, in *E.coli,* wobei die Linkersequenz einer der Sequenzen SEQ ID NOs 2, 4, 6, 8, 10, 13, 14, 20 oder 63 entspricht,
- Spaltung des Fusionsproteins durch eine geeignete Caspase für die Erkennungsstelle der Caspase Gruppe II, die spezifisch das Fusionsprotein an der Verbindung der Linkersequenz und dem aminoterminalen Aminosäurerestes des reifen Polypeptides von Interesse spaltet, und
- Isolierung des biologisch aktiven Polypeptides von Interesse.

**16.** Ein Verfahren zur Herstellung eines Polypeptides von Interesse in einer biologisch aktiven Form, umfassend:

- Herstellung in E.coli (1) eines Fusionsprotein umfassend ein aminoterminales Polypeptid, eine Linkersequenz umfassend eine Erkennungsstelle der Caspase Gruppe II, den Polypeptides von Interesse, und (2) eines geeigneten Caspase-Proteins für die Erkennungsstelle der Caspase Gruppe II, wobei das Fusionsprotein und das Caspase-Polypeptid von dem gleichen oder einem unterschiedlichen Promoter kontrolliert werden,
- Induzierung der Expression des Fusionsproteins
- Induzierung der Expression des Caspase-Proteins
- Spaltung des Fusionsproteins durch das Caspase-Protein in vivo, wobei die Caspase das Fusionsprotein an der Verbindungsstelle der Linkersequenz und des aminoterminalen Aminosäurerest des reifen Polypeptides von Interesse spaltet, und
- Isolierung des biologisch aktiven Polypeptides von Interesse.

**17.** Das Verfahren nach Anspruch 16, wobei die Aminosäuresequenz des Linkers einer der Sequenzen SEQ ID NOs 2, 4, 6, 8, 10, 13, 14, 20 oder 63 entspricht.

**18.** Das Verfahren nach einem der Ansprüche 15 bis 17, wobei das Caspase-Protein Caspase-3 oder Caspase-7 ist.

**19.** Das Verfahren nach einem der Ansprüche 16 bis 18, wobei eine einzelne Polynukleinsäure das Fusionsprotein und die Caspase kodiert.

**20.** Das Verfahren nach einem der Ansprüche 16 bis 18, wobei unterschiedliche Polynukleinsäuren das Fusionsprotein und die Caspase kodieren.

**21.** Eine Polynukleinsäure kodierend ein Caspase-Protein und kodierend ein Fusionsprotein umfassend ein aminoterminales Polypeptid, ein reifes Polypeptid von Interesse und eine Linkersequenz kodierend ein Peptid einer der Sequenzen SEQ ID NOs 2, 4, 6, 8, 10 zwischen dem aminoterminalen Polypeptid und dem reifen Polypeptid von Interesse, wobei die Linkersequenz eine Erkennungsstelle der Caspase Gruppe II an der Verbindung zwischen dem Linker und der aminoterminalen Aminosäure des reifen Polypeptides von Interesse umfasst.

**22.** Die Polynukleinsäure nach Anspruch 21, wobei das Caspase-Protein aus der Gruppe, bestehend aus Caspase-2, Caspase-3, Caspase-7, CED-3, reverse Caspase-3 und reverse Caspase-7, ausgewählt ist.

**23.** Die Polynukleinsäure nach Anspruch 21 oder 22, wobei das aminoterminale Polypeptid aus der Gruppe, bestehend aus Glutathion-S-Transferase Gen GST, E.coli Thioredoxin TRX, NusA, Chitin-Bindedomäne CBD, Chloramphenicolacetyltransferase CAT, Protein A (prot A), LysRS, Maltose-Bindungsprotein (MBP), Ubiquitin, Calmodulin, DsbA, DsbC und Lambda gpV, ausgewählt ist.

**24.** Die Polynukleinsäure nach einem der Ansprüche 21 bis 23, wobei die Linkersequenz mindestens 5 Aminosäuren umfasst.

**25.** Die Polynukleinsäure nach einem der Ansprüche 21 bis 24, wobei die Caspase-Erkennungsstelle aus der Aminosäuresequenz DELD, DXXD, DEVD oder DEHD besteht.

**26.** Die Polynukleinsäure nach einem der Ansprüche 21 bis 25, wobei die Expression des Fusionsproteins und/oder des Caspase-Proteins von einem geeigneten Promoter kontrolliert wird.

**27.** Die Polynukleinsäure nach Anspruch 26, wobei das Fusionsprotein und das Caspase-Protein von dem gleichen oder einem unterschiedlichen Promoter kontrolliert werden.

**28.** Die Polynukleinsäure nach Anspruch 26 oder 27, wobei der Promoter induzierbar ist.

**29.** Die Polynukleinsäure nach einem der Ansprüche 21 bis 28, wobei das Fusionsprotein ferner einen Detektions- oder Affinitäts-Tag umfasst.

**30.** Die Polynukleinsäure nach Anspruch 29, wobei der Detektions- oder Affinitäts-Tag aus der Gruppe, bestehend aus Polyhistidin-Tag, Polyarginin-Tag, Streptavidin-Bindungs-Tag, biotinylierten Tag und Tag, der von einem Antikörper erkannt wird, ausgewählt ist.

**31.** Ein Vektor, der die Polynukleinsäure nach einem der Ansprüche 21 bis 30 umfasst.

**32.** Eine Wirtszelle, die die Polynukleinsäure nach einem der Ansprüche 21 bis 30 oder den Vektor nach Anspruch 31 umfasst.

**33.** Ein Fusionsprotein, das durch die Nukleinsäure nach einem der Ansprüche 21 bis 30 kodiert wird.

**34.** Verwendung einer Nukleinsäure nach einem der Ansprüche 21 bis 30, einem Vektor nach Anspruch 31 oder einer Wirtszelle nach Anspruch 32 für die Herstellung eines Polypeptides von Interesse in einer reifen und/oder biologisch aktiven Form.

**35.** Ein geeigneter Bioreaktor für die Herstellung von reifen Proteinen von Interesse, umfassend:

(a) ein Fusionsprotein, das von einer Polynukleinsäure nach Anspruch 29 kodiert wird, wobei das Fusionsprotein einen Affinitäts-Tag mit einer spezifischen Affinität für ein Substrat umfasst, und
(b) ein Substrat, wobei der Teil des Affinitäts-Tags des Fusionsproteins an das Substrat gebunden ist.

**36.** Ein geeigneter Bioreaktor für die Herstellung von reifen Proteinen von Interesse, umfassend:

(a) ein Fusionsprotein, das von einer Polynukleinsäure nach Anspruch 29 kodiert wird, wobei das Fusionsprotein einen Affinitäts-Tag mit einer spezifischen Affinität für ein Substrat umfasst, und
(b) einem Caspase-Enzym gekoppelt an oder mit Affinität für einen festen Träger, wobei das Caspase-Enzym spezifisch die Caspase-Erkennungsstelle in dem Fusionsprotein spaltet.

**37.** Ein Verfahren zur Herstellung eines Proteins von Interesse, umfassend:

(a) Herstellung eines Fusionsproteins nach Anspruch 29, wobei das Fusionsprotein einen Affinitäts-Tag oder ein aminoterminales Polypeptid mit einer spezifischen Affinität für ein Substrat umfasst,
(b) Einsetzen des Fusionsproteins in einen Bioreaktor umfassend ein Caspase-Enzym, an das ein fester Träger gekoppelt ist,
(c) Inkubieren des Fusionsproteins mit dem Caspase-Enzym während einer geeigneten Inkubationszeit, und
(d) Waschen des Reaktors und Einfangen des eluierten Fusionsproteins durch die Bindung an das Substrat.

**Revendications**

**1.** Procédé de production d'un polypeptide d'intérêt sous une forme biologiquement active, comprenant :

la production, dans un hôte approprié, d'une protéine de fusion comprenant un polypeptide à extrémité aminé, une séquence de liaison comprenant un site de reconnaissance de caspase de groupe II et le polypeptide d'intérêt,
le clivage de ladite protéine de fusion par une caspase capable de reconnaître ledit site de reconnaissance de caspase de groupe II, qui clive spécifiquement la protéine de fusion à la jonction de la séquence de liaison et du résidu d'acide aminé de l'extrémité aminé du polypeptide mature d'intérêt, et
- l'isolement du polypeptide d'intérêt biologiquement actif.

2. Procédé selon la revendication 1, dans lequel ledit hôte approprié est un procaryote.

3. Procédé selon la revendication 2, dans lequel ledit procaryote est *Escherichia coli.*

4. Procédé selon l'une quelconque des revendications 1 à 3, dans lequel ledit site de reconnaissance de caspase comprend la séquence d'acides aminés DELD, DXXD, DEVD ou DEHD.

5. Procédé selon l'une quelconque des revendications 1 à 4, dans lequel ladite caspase est choisie dans le groupe consistant en caspase-2, caspase-3, caspase-7, CED-3, caspase-3 inverse et caspase-7 inverse.

6. Procédé selon l'une quelconque des revendications 1 à 5, dans lequel ladite séquence de liaison comprend au moins 5 acides aminés.

7. Procédé selon l'une quelconque des revendications 1 à 6, dans lequel ladite séquence de liaison est représentée par l'une quelconque des SEQ ID NO 2, 4, 6, 8, 10, 13, 14, 20 ou 63.

8. Procédé selon l'une quelconque des revendications 1 à 7, dans lequel ledit clivage de la protéine de fusion par la caspase est effectué *in vitro.*

9. Procédé selon l'une quelconque des revendications 1 à 8, dans lequel un rendement d'au moins 80% de polypeptide mature d'intérêt est obtenu en 60 minutes comme cela est estimé par balayage densitométrique.

10. Procédé selon l'une quelconque des revendications 1 à 7, **caractérisé de plus en ce que** la protéine caspase est produite dans le même hôte que la protéine de fusion.

11. Procédé selon l'une quelconque des revendications 1 à 7, **caractérisé de plus en ce que** la séquence de protéine caspase est comprise dans la protéine de fusion.

12. Procédé selon la revendication 10 ou 11, **caractérisé de plus en ce que** la production de la protéine caspase et de la protéine de fusion est successive.

13. Procédé selon la revendication 12, **caractérisé en ce que** la protéine caspase est produite après la protéine de fusion.

14. Procédé selon l'une quelconque des revendications 9 à 13, dans lequel ledit clivage de la protéine de fusion est effectué *in vivo.*

15. Procédé de production d'un polypeptide d'intérêt sous une forme biologiquement active, comprenant :

- la production, dans *E. coli,* d'une protéine de fusion comprenant un polypeptide à extrémité amino, une séquence de liaison comprenant un site de reconnaissance de caspase de groupe II et le polypeptide d'intérêt, où la séquence de liaison est représentée par l'une quelconque des SEQ ID NO 2, 4, 6, 8, 10, 13, 14, 20 ou 63,
- le clivage de ladite protéine de fusion par une caspase capable de reconnaître ledit site de reconnaissance de caspase de groupe II, qui clive spécifiquement la protéine de fusion à la jonction de la séquence de liaison et du résidu d'acide aminé de l'extrémité amino du polypeptide mature d'intérêt, et
- l'isolement du polypeptide d'intérêt biologiquement actif.

16. Procédé de production d'un polypeptide d'intérêt sous une forme biologiquement active, comprenant :

- la production, dans *E. coli,* (1) d'une protéine de fusion comprenant un polypeptide à extrémité aminé, une séquence de liaison comprenant un site de reconnaissance de caspase de groupe II, le polypeptide d'intérêt et (2) d'une protéine caspase, capable de reconnaître ledit site de reconnaissance de caspase de groupe II, où ladite protéine de fusion et ledit polypeptide caspase sont sous le contrôle d'un promoteur identique ou différent,
- l'induction de l'expression de la protéine de fusion,

- l'induction de l'expression de la protéine caspase,
- le clivage de ladite protéine de fusion *in vivo* par la protéine caspase qui clive la protéine de fusion à la jonction de la séquence de liaison et du résidu d'acide aminé de l'extrémité aminé du polypeptide mature d'intérêt, et
- l'isolement du polypeptide d'intérêt biologiquement actif.

17. Procédé selon la revendication 16, dans lequel la séquence d'acides aminés de la séquence de liaison est représentée par l'une quelconque des SEQ ID NO 2, 4, 6, 8, 10, 13, 14, 20 ou 63.

18. Procédé selon l'une quelconque des revendications 15 à 17, dans lequel ladite protéine caspase est la caspase-3 ou la caspase-7.

19. Procédé selon l'une quelconque des revendications 16 à 18, dans lequel un unique acide polynucléique code la protéine de fusion et la caspase.

20. Procédé selon l'une quelconque des revendications 16 à 18, dans lequel des acides polynucléiques séparés codent la protéine de fusion et la caspase.

21. Acide polynucléique codant une protéine caspase et codant une protéine de fusion comprenant un polypeptide à extrémité aminé, un polypeptide mature d'intérêt et une séquence de liaison codant un peptide représenté par l'une quelconque des SEQ ID No 2, 4, 6, 8, 10, entre le polypeptide à extrémité aminé et le polypeptide mature d'intérêt, ladite séquence de liaison comprenant un site de reconnaissance de caspase de groupe II à la jonction de la séquence de liaison et de l'acide aminé de l'extrémité aminé du polypeptide mature d'intérêt.

22. Acide polynucléique selon la revendication 21, dans lequel ladite protéine caspase est choisie dans le groupe consistant en caspase-2, caspase-3, caspase-7, CED-3, caspase-3 inverse et caspase-7 inverse.

23. Acide polynucléique selon les revendications 21 ou 22, dans lequel ledit polypeptide à extrémité aminé est choisi dans le groupe consistant en gène GST de la glutathion S-transférase, thiorédoxine TRX de E. coli, NusA, domaine CBD de liaison de la chitine, chloramphénicol acétyl transférase CAT, protéine A (prot A), LysRS, protéine de liaison du maltose (MBP), ubiquitine, calmoduline, DsbA, DsbC et lambda gpV.

24. Acide polynucléique selon l'une quelconque des revendications 21 à 23, dans lequel ladite séquence de liaison comprend au moins 5 acides aminés.

25. Acide polynucléique selon l'une quelconque des revendications 21 à 24, dans lequel ledit site de reconnaissance de caspase consiste en la séquence d'acides aminés DELD, DXXD, DEVD ou DEHD.

26. Acide polynucléique selon l'une quelconque des revendications 21 à 25, dans lequel l'expression de ladite protéine de fusion et/ou de la protéine caspase est sous le contrôle d'un promoteur approprié.

27. Acide polynucléique selon la revendication 26, dans lequel la protéine de fusion et la protéine caspase sont sous le contrôle d'un promoteur identique ou différent.

28. Acide polynucléique selon les revendications 26 ou 27, dans lequel ledit promoteur est un promoteur inductible.

29. Acide polynucléique selon l'une quelconque des revendications 21 à 28, dans lequel ladite protéine de fusion comprend de plus un marqueur de détection ou d'affinité.

30. Acide polynucléique selon la revendication 29, dans lequel ledit marqueur de détection ou d'affinité est choisi dans le groupe consistant en un marqueur polyhistidine, un marqueur polyarginine, un marqueur de liaison streptavidine, un marqueur biotinylé et un marqueur reconnu par un anticorps.

31. Vecteur comprenant l'acide polynucléique selon l'une quelconque des revendications 21 à 30.

32. Cellule hôte comprenant l'acide polynucléique selon l'une quelconque des revendications 21 à 30 ou comprenant le vecteur selon la revendication 31.

33. Protéine de fusion codée par l'acide nucléique selon l'une quelconque des revendications 21 à 30.

EP 1 597 369 B1

34. Utilisation d'un acide polynucléique selon l'une quelconque des revendications 21 à 30, d'un vecteur selon la revendication 31 ou d'une cellule hôte selon la revendication 32 pour la production d'un polypeptide d'intérêt sous une forme mature et/ou biologiquement active.

35. Bioréacteur adapté à la production de protéines matures d'intérêt comprenant :

(a) une protéine de fusion codée par un acide polynucléique selon la revendication 29, ladite protéine de fusion comprenant un marqueur d'affinité ayant une affinité spécifique pour un substrat, et
(b) un substrat,

dans lequel la partie marqueur d'affinité de la protéine de fusion est liée au substrat.

36. Bioréacteur adapté à la production de protéines matures d'intérêt comprenant :

(a) une protéine de fusion codée par un acide polynucléique selon la revendication 29, ladite protéine de fusion comprenant un marqueur d'affinité ayant une affinité spécifique pour un substrat, et
(b) une enzyme caspase couplée à un support solide ou ayant une affinité pour celui-ci,

dans lequel l'enzyme caspase clive spécifiquement le site de reconnaissance de caspase dans la protéine de fusion.

37. Procédé de production d'une protéine d'intérêt, comprenant :

(a) la production d'une protéine de fusion selon la revendication 29, ladite protéine de fusion comprenant un marqueur d'affinité ou un polypeptide à extrémité aminé ayant une affinité spécifique pour un substrat,
(b) l'introduction de ladite protéine de fusion dans un bioréacteur comprenant une enzyme caspase couplée à un support solide,
(c) l'incubation de ladite protéine de fusion avec l'enzyme caspase pendant une période d'incubation appropriée, et
(d) le lavage du réacteur et la capture de la protéine de fusion éluée par liaison au substrat.

Fig. 1:

Group I    Hydrophobic
Group II         Asp    Glu    Xaa    Asp
Group III   Aliphatic

Fig. 2

EcoRV

SEQ ID

Fusion partner F

**1** GGG CCC GGC TCG | GAC GAA GTG GAT | ATC — SEQ ID 1
G   P   G   S      D   E   V   D — SEQ ID 2

**2** F — GGG CCC GGC TCG GGA GGG GGA TCA GGT | GAC GAA GTG GAT | ATC — SEQ ID 3
G   P   G   S   G   G   G   S   G      D   E   V   D — SEQ ID 4

**3** F — GGG CCC GGC TCG CAT CAT CAC CAT GGA | GAC GAA GTG GAT | ATC — SEQ ID 5
G   P   G   S   H   H   H   H   G      D   E   V   D — SEQ ID 6

**4** F — GGG CCC GGC TCG CAT CAC CAC CAT AAG GAC GAT | GAC GAA GTG GAT | ATC — SEQ ID 7
G   P   G   S   H   H   H   H   K   D   D      D   E   V   D — SEQ ID 8

**5** F — GGG CCC GAT GAC GGC GAA GGA GAG AAG GAC GAT | GAC GAA GTG GAT | ATC — SEQ ID 9
G   P   D   D   G   E   G   E   K   D   D      D   E   V   D — SEQ ID 10

Fig. 3A

Fig. 3B

Fig. 4:

EP 1 597 369 B1

H-GST     mLIF

F     L     P

Thrombin:   RGPR/
Caspase-3: DEVD/
Caspase-3: DEVD/S

**Thrombin**     **Caspase-3**

**DEVD/S**     **DEVD/**

22°C     4°C     45' 30°C     45' 30°C

units  |0.2||0.4|| 1 ||0.2||0.4|| 1 |  ng | 0 || 50||125||250||500||1000||1000|     | 0 || 50||125||250||500||1000||1000|

GST-mLIF ▶     61
Thrombin ▶     49
36
GST ▶
mLIF ▶     C3 ▷     25
19
Thrombin cut 16h     35     13

36

Fig. 5

Fig. 6A:

Fig. 6B

Fig. 6C

Fig. 7

mLIF          hIFNα

C3      C7       devd

|devd||devds| |devd||devds|      | C3 || C7 |

61
49
36
GST                                          25      GST
mLIF                                         19      Casp7
                                                     hIFNα
13      Casp7

## mLIF

Fig. 8:

MGSPLPITPVNATCAIRHPCHGNLMNQIKNQLAQLNGSANALFISYYTAQGEPFPNNVEK
LCAPNMTDFPSFHGNGTEKTKLVELYRMVAYLSASLTNITRDQKVLNPTAVSLQVKLNAT
IDVMRGLLSNVLCRLCNKYRVGHVDVPPVP DHSD KEAFQRKKLGCQLLGTYKQVISVVVQ
AF                                                    SEQ ID 59

## hIFNa

MCDLPQTHSLGSRRTLMLLAQMRRISLFSCLK DRHD FGFPQEEFGNQFQKAETIPVLHEM
IQQIFNLFSTKDSSAAWDETLLDKFYTELYQQLNDLEACVIQGVGVTETPLMKEDSILAV
RKYFQRITLYLKEKKYSPCAWEVVRAEIMRSFSLSTNLQESLRSKE          SEQ ID 60

## GST

SPILGYWKIKGLVQPTRLLLEYLEEKYEEHLYER DEGD KWRNKKFELGLEFPNLPYYIDG
DVKLTQSMAIIRYIADKHNMLGGCPKERAEISMLEGAVLDIRYGVSRIAYSKDFETLKVD
FLSKLPEMLKMFEDRLCHKTYLNGDHVTHPDFMLY DALD VVLYMDPMCLDAFPKLVCFKK
RIEAIPQIDKYLKSSKYIAWPLQGWQATFGGGDHPPKSDL                SEQ ID 61

## TRX

SDKIIHLTD DSFD TDVLKADGAILVDFWAEWCGPCKMIAPILDEIADEYQGKLTVAKLNI
DQNPGTAPKYGIRGIPTLLLFKNGEVAATKVGALSKGQLKEFLDANLAGS      SEQ ID 62

Fig. 9:

Silverstain 2µg

Fig. 10A

pICA11
9269 bps

KmR
mCasp3p30
pRpM
cl857
ant
Ptac
lacI
Placlq
repA

pICA12
10603 bps

laclQ
pRpM
cl857
ant
Ptac
KmR
repA
mCasp3p30
Para
araC

Fig. 10B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0061768 A
- EP 1013763 A1
- WO 0206458 A
- WO 0073437 A
- US 6207420 B
- WO 9848025 A
- US 26379950 B
- PT 102705

- US 6379950 B
- WO 9213955 A
- WO 9319091 A
- WO 8809372 A
- US 5665566 A
- US 5532142 A
- EP 03447034 A


**Non-patent literature cited in the description**

- **GU et al.** *Science,* 1997, vol. 275, 206-209
- **COLUSSI, P. A. ; N. L. HARVEY ; L. M. SHEARWIN-WHYATT ; S. KUMAR.** Conversion of procaspase-3 to an autoactivating caspase by fusion to the caspase-2 prodomain. *J Biol Chem,* 1998, vol. 273 (41), 26566-70
- **GARCIA-CALVO, M. ; E. P. PETERSON ; D. M. RASPER ; J. P. VAILLANCOURT ; R. ZAMBONI ; D. W. NICHOLSON ; N. A. THORNBERRY.** Purification and catalytic properties of human caspase family members. *Cell Death Differ,* 1999, vol. 6 (4), 362-9
- **NICHOLSON, D. W.** Caspase structure, proteolytic substrates, and function during apoptotic cell death. *Cell Death Differ,* 1999, vol. 6 (11), 1028-42
- **NICHOLSON, D. W. ; N. A. THORNBERRY.** Caspases: killer proteases. *Trends Biochem Sci,* 1997, vol. 22 (8), 299-306
- **STENNICKE, H. R. ; M. RENATUS ; M. MELDAL ; G. S. SALVESEN.** Internally quenched fluorescent peptide substrates disclose the subsite preferences of human caspases 1, 3, 6, 7 and 8. *Biochem J,* 2000, vol. 350 (2), 563-8
- **STENNICKE, H. R. ; G. S. SALVESEN.** Biochemical characteristics of caspases-3, -6, - 7, and -8. *J Biol Chem,* 1997, vol. 272 (41), 25719-23

- **STENNICKE, H. R. ; G. S. SALVESEN.** Catalytic properties of the caspases. *Cell Death Differ,* 1999, vol. 6 (11), 1054-9
- **THORNBERRY, N. A. ; K. T. CHAPMAN ; D. W. NICHOLSON.** Determination of caspase specificities using a peptide combinatorial library. *Methods Enzymol,* 2000, vol. 322, 100-10
- **VAN DE CRAEN, M. ; W. DECLERCQ ; I. VAN DEN BRANDE ; W. FIERS ; P. VANDENABEELE.** The proteolytic procaspase activation network: an in vitro analysis. *Cell Death Differ,* 1999, vol. 6 (11), 1117-24
- **ALNEMRI, E.** *Recombinant, active caspases and uses thereof,* 1999
- **MCCOY, J. ; E. LAVALLIE.** *Peptide and protein fusions to thioredoxin and thioredoxin-like molecules,* 1993
- **SMITH, D.** *Tripartide fusion proteins of gluthathione S-transferase,* 1993
- **SMITH, D.** *Novel fusion proteins containing gluthathione S-transferase,* 1988
- **LAVALLIE, E.** *Cloning of Enterokinase and method of use,* 1994
- **JOHNSTON, S. ; W. DOUGHERTY.** *Method of isolation and purification of fusion polypeptides,* 1993